# EUROPÄISCHE PATENTSCHRIFT

(11) **EP 2 337 553 B1**
(45) Veröffentlichungstag und Bekanntmachung des Hinweises auf die Patenterteilung: **04.04.2018**
(21) Anmeldenummer: 09780001.5
(22) Anmeldetag: 29.06.2009
(51) Int. Cl.: A61K 8/81, A61K 8/891, A61K 8/898, A61Q 5/06, C08F 220/06

(54) **GESCHMEIDIGES STYLINGMITTEL MIT HOHEM HALTEGRAD**
SMOOTH STYLING AGENTS GIVING A HIGH DEGREE OF HOLD
PRODUIT COIFFANT SOUPLE ASSURANT UNE TENUE FORTE

(30) Priorität: 21.07.2008 DE 102008034102
(43) Veröffentlichungstag der Anmeldung: 29.06.2011
(73) Patentinhaber: Henkel AG & Co. KGaA, 40589 Düsseldorf (DE)
(72) Erfinder: KNAPPE, Thorsten, 22869 Schenefeld (DE); FLODROP VAN, Helga, 21075 Hamburg (DE)
(86) Internationale Anmeldenummer: PCT/EP2009/058106
(87) Internationale Veröffentlichungsnummer: WO 2010/009953

(56) Entgegenhaltungen:
- EP-A1- 1 726 600
- EP-A2- 0 470 381
- EP-A2- 0 479 000
- WO-A2-2006/018328
- WO-A2-2009/016017
- FR-A1- 2 681 245
- US-A- 6 149 898
- T.E. Gottschalck, G.N. McEwen, Jr.: "International Cosmetic Ingredient Dictionary and Handbook", 2006, The Cosmetic, Toiletry and Fragrance Association, Washington, D.C., XP002619200, ISBN: 1-882621-36-0 Bd. 2, Seite 2161, Spalte 3

## Beschreibung

Die vorliegende Erfindung betrifft Mittel zur temporären Verformung keratinischer Fasern, enthaltend eine Kombination von Polymeren mit weiteren speziellen Inhaltstoffen, die Verwendung dieser Mittel zur temporären Verformung keratinischer Fasern und ein entsprechendes Verfahren.

Unter keratinischen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Eine ansprechend aussehende Frisur wird heute allgemein als unverzichtbarer Teil eines gepflegten Äußeren angesehen. Dabei gelten aufgrund von aktuellen Modeströmungen immer wieder Frisuren als chic, die sich bei vielen Haartypen nur unter Verwendung festigender Wirkstoffe aufbauen bzw. für einen längeren Zeitraum bis hin zu mehreren Tagen aufrechterhalten lassen. Daher spielen Haarbehandlungsmittel, die einer permanenten oder temporären Formgebung der Haare dienen, eine wichtige Rolle. Temporäre Formgebungen, die einen guten Halt ergeben sollen, ohne das gesunde Aussehen der Haare, wie zum Beispiel deren Glanz, zu beeinträchtigen, können beispielsweise durch Haarsprays, Haarwachse, Haargele, Haarschäume, Fönwellen etc. erzielt werden.

Entsprechende Mittel zur temporären Formgebung enthalten als formgebende Komponente üblicherweise synthetische Polymere. Zubereitungen, die ein gelöstes oder dispergiertes Polymer enthalten, können mittels Treibgasen oder durch einen Pumpmechanismus auf das Haar aufgebracht werden. Insbesondere Haargele und Haarwachse werden allerdings in der Regel nicht direkt auf das Haar appliziert, sondern mittels eines Kamms oder der Hände im Haar verteilt.

Die wichtigste Eigenschaft eines Mittels zur temporären Verformung keratinischer Fasern, im Folgenden auch Stylingmittel genannt, besteht darin, den behandelten Fasern in der erzeugten Form einen möglichst starken Halt zu geben. Handelt es sich bei den keratinischen Fasern um menschliche Haare, spricht man auch von starkem Frisurenhalt oder vom hohen Haltegrad des Stylingmittels. Der Frisurenhalt wird im Wesentlichen durch die Art und Menge des eingesetzten synthetischen Polymers bestimmt, wobei jedoch auch ein Einfluss der weiteren Bestandteile des Stylingmittels gegeben sein kann.

Neben einem hohen Haltegrad müssen Stylingmittel eine ganze Reihe weiterer Anforderungen erfüllen. Diese können grob in Eigenschaften am Haar, Eigenschaften der jeweiligen Formulierung, z.B. Eigenschaften des Schaums, des Gels oder des versprühten Aerosols, und Eigenschaften, die die Handhabung des Stylingmittels betreffen, unterteilt werden, wobei den Eigenschaften am Haar besondere Wichtigkeit zukommt. Zu nennen sind insbesondere Feuchtebeständigkeit, niedrige Klebrigkeit und ein ausgewogener Konditioniereffekt. Weiterhin soll ein Stylingmittel möglichst für alle Haartypen universell einsetzbar sein. Oft geht ein hoher Haltegrad unerwünschterweise mit einer hohen Sprödigkeit der Frisur einher. Die mit dem entsprechenden Stylingmittel behandelten Haar sind starr, spröde und wirken unnatürlich fest. Dadurch fühlen sie sich rauh und ungepflegt an. Zudem ist der Polymerfilm, den die Mittel bei der Anwendung auf dem Haar hinterlassen in den genannten Fällen so unflexibel, daß er bei Beanspruchung bricht. Hierdurch kommt es zur Bildung so genannter Filmplaken, das heißt Rückständen, die sich bei der Bewegung des Haares ablösen und den Eindruck vermitteln, der Anwender des entsprechenden Stylingmittels hätte Schuppen. Ein weiteres Problem besteht darin, daß die Produktkonsistenz solcher Produkte vom Verbraucher negativ beurteilt wird, indem diese Produkte als zäh, klebrig und schlecht applizierbar angesehen werden.

Aufgabe der vorliegenden Erfindung war es daher, ein Mittel zur temporären Verformung keratinischer Fasern zur Verfügung zu stellen, das sich durch einen sehr hohen Haltegrad auszeichnet ohne daß dabei auf Flexibilität und ein gepflegtes Haargefühl sowie angenehme Produkthaptik verzichtet werden müsste.

Es wurde nunmehr überraschenderweise gefunden, daß sich Stylingprodukte mit einem hohen Haltegrad und einem angenehmem Pflegegefühl im Haar bereitstellen lassen, indem eine Kombination spezieller Polymere mit Silikonen in die Mittel inkorporiert wird.

Ein erster Gegenstand der vorliegenden Erfindung ist daher die Verwendung eines kosmetischen Mittels, enthaltend in einem kosmetisch akzeptablen Träger
a) mindestens ein Copolymer A, das mindestens eine Struktureinheit gemäß Formel (I) und mindestens eine Struktureinheit gemäß Formel (II) und mindestens eine Struktureinheit gemäß Formel (III) enthält 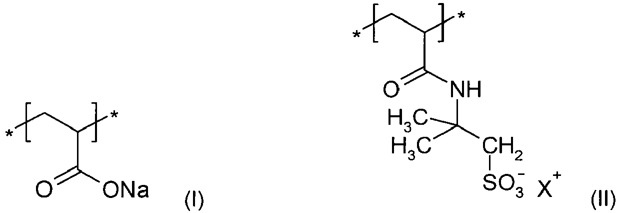 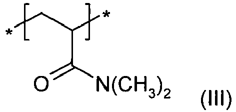 worin X⁺ für ein physiologisch verträgliches Kation steht und
b) mindestens ein Silikonöl und/oder Silikongum
   zur temporären Verformung keratinischer Fasern.

Die internationale Patentanmeldung WO 2006/018328 A2 beschreibt kosmetische Haarverformungsmittel, die neben Silikonen auch Copolymere aus Acrylamid und 2-Acrylamido-2-methylpropansulfonsäure enthalten können.
Haarkosmetische Mittel auf Grundlage von Silikonöl oder Silikongummi werden in den
Patentanmeldungen FR 2 681 245 und US 6,149,898 beschrieben.
In der internationalen Anmeldung WO 2009/0916017 werden Copolymerisate aus Natriumacrylat, Natriumacryloyldimethyltaurat und Dimethylacrylamid offenbart.

Die erfindungsgemäßen Mittel enthalten als erste zwingende Komponente ein Polymer, welches aus mindestens drei verschiedenen Monomeren der Formeln (I), (II), und (III) aufgebaut ist. Darüber hinaus können weitere Monomere einpolymerisiert sein.

Das erste Monomer, das im Copolymer A enthalten ist, ist Natriumacrylat, d.h. das Natriumsalz der Acrylsäure. Zusätzlich hierzu kann auch noch Acrylsäure als weiterer Monomerbaustein in den Polymeren vorliegen, dies ist erfindungsgemäß aber nicht zwingend notwendig.

Das zweite Monomer, das im Copolymer A enthalten ist, ist 2-Methyl-2[(1-oxo-2-propenyl)amino]-1-propansulfonsäure (AMPS), die teilweise oder vollständig neutralisiert vorliegen kann. Üblicherweise sind als Kationen Na⁺ und NH₄⁺ bevorzugt.

Das dritte Monomer, das im Copolymer A enthalten ist, ist Dimethylacrylamid. Die Copolymere A lassen sich durch die allgemeine Formel 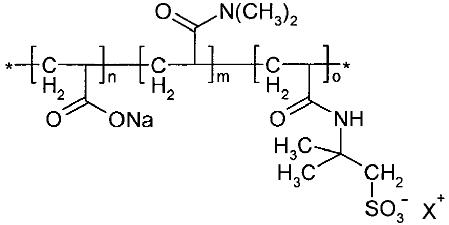 beschreiben, wobei die Indices m und n und o je nach Molmasse des Polymers variieren und nicht bedeuten sollen, daß es sich zwingend um Blockcopolymere handelt. Vielmehr können Struktureinheiten der Formeln (I), (II) und (III) im Molekül auch statistisch verteilt vorliegen.

Die Monomere der Formeln (I), (II) und (III) sind in den Copolymeren A vorzugsweise in solcher Anzahl und Verteilung enthalten, das das Copolymer A Molmassen zwischen 5 und 1000 kDa aufweist. Bevorzugte erfindungsgemäße Mittel sind dadurch gekennzeichnet, daß sie Copolymer(e) A mit Molmassen von 10 bis 750 kDa, vorzugsweise von 25 bis 500 kDa, weiter bevorzugt von 50 bis 400 kDa und insbesondere von 70 bis 250 kDa, enthalten.

Vorzugsweise sind die Monomere der Formeln (I), (II) und (III) innerhalb bestimmter Grenzen im Copolymer A enthalten. Hier sind erfindungsgemäß bevorzugte Mittel dadurch gekennzeichnet, daß sie Copolymer(e) A enthalten, die
- 10 bis 90 Mol.-%, vorzugsweise 15 bis 85 Mol.-% und insbesondere 20 bis 80 Mol.-% Monomere der Formel (I) und
- 5 bis 85 Mol.-%, vorzugsweise 7,5 bis 80 Mol.-% und insbesondere 10 bis 60 Mol.-% Monomere der Formel (II) und
- 5 bis 85 Mol.-%, vorzugsweise 10 bis 80 Mol.-% und insbesondere 15 bis 70 Mol.-% Monomere der Formel (III)
enthalten.

Unabhängig davon, ob die erfindungsgemäßen Mittel ein oder mehrere Copolymere A enthalten, ist es bevorzugt, die Copolymere A innerhalb bestimmter Mengenbereiche einzusetzen. Hier sind erfindungsgemäße Mittel bevorzugt, die, bezogen auf das Gewicht des anwendungsbereiten Mittels, 0,1 bis 10 Gew.-%, vorzugsweise 0,5 bis 7,5 Gew.-% und insbesondere 1 bis 5 Gew.-% Copolymer(e) A enthalten.

Ganz besonders bevorzugt enthalten die erfindungsgemäßen Zusammensetzungen das Copolymer A in Form eines inversen, auto-inversiblen Polymerlatex, enthaltend eine Ölphase, eine Wasserphase, mindestens einen Öl-in-Wasser-Emulgator und mindestens einen verzweigten oder vernetzten Polyelektrolyten (= Copolymer A), der ein Copolymer darstellt, das mindestens ein Monomer mit einer starken Säurefunktion sowie mindestens ein weiteres Monomer aufweist, das neutral ist oder eine schwache Säurefunktion enthält.

Allen vorgenannten, bevorzugt verwendeten inversen Polymerlatices ist gemein, dass sie mindestens ein Öl, bevorzugt ausgewählt aus weißen Mineralölen, Squalan, Isohexadecan und (gegebenenfalls hydriertem) Polyisobuten, sowie mindestens einen Öl-in-Wasser-Emulgator, ausgewählt aus den Ethylenoxid-Addukten von Sorbitanoleat, Ricinusöl, das gewünschtenfalls gehärtet ist, Sorbitanlaurat und Laurylalkohol, sowie weiterhin ausgewählt aus der Polyethylenoxid-freien Öl-in-Wasser-Emulgatorklasse der C6-C22-Fettalkohol-Glucoseether, insbesondere Caprinic Glucoside, Caprylic Glucoside, Capric Glucoside, Lauric Glucoside, Myristic Glucoside, Cetyl Glucoside, Stearyl Glucoside, Arachidyl Glucoside, Behenyl Glucoside, besonders bevorzugt Caprylic Glucoside und Capric Glucoside, enthalten. Demnach sind bevorzugte erfindungsgemäße Zusammensetzungen dadurch gekennzeichnet, dass die Ölphase des inversen Polymerlatex mindestens ein Öl, ausgewählt aus weißen Mineralölen, Squalan, Isohexadecan und (gegebenenfalls hydriertem) Polyisobuten, enthält. Weiter bevorzugte erfindungsgemäße Zusammensetzungen sind dadurch gekennzeichnet, dass der in dem inversen Polymerlatex enthaltene Öl-in-Wasser-Emulgator ausgewählt ist aus den Ethylenoxid-Addukten von Sorbitanoleat, Ricinusöl, das gewünschtenfalls gehärtet ist, Sorbitanlaurat und Laurylalkohol, sowie aus Caprylic Glucoside und Capric Glucoside.

Die erfindungsgemäß bevorzugten einzusetzenden verdickenden Polymerlatices weisen vorzugsweise einen Polymergehalt an Copolymer A von 30 - 90 Gew.-%, bevorzugt 35 - 75 Gew.-% und besonders bevorzugt 40 - 60 Gew.-%, jeweils bezogen auf den gesamten Latex, auf. Der Polymergehalt des Latex hat dabei jedoch eher nur eine Bedeutung für die Herstellung der erfindungsgemäßen Zusammensetzungen, z. B. in Bezug auf die Mischbarkeit bzw. Dosierbarkeit. Für die erfindungsgemäßen Zusammensetzungen selbst ist eher der Polymergehalt an sich, bezogen auf die erfindungsgemäße Zusammensetzung, bedeutsam, siehe oben.

Besonders bevorzugt wird das Copolymer A erfindungsgemäß in Form eines inversen, auto-inversiblen Polymerlatex eingesetzt, der zusätzlich zum Copolymer A Isohexadecan als Ölphase und Sorbitanmonostearat als Emulgator enthält.

Als zweiten wesentlichen Inhaltsstoff enthalten die erfindungsgemäßen Mittel mindestens ein Silikonöl und/oder Silikongum. Erfindungsgemäße bevorzugte Mittel sind dadurch gekennzeichnet, daß sie mindestens ein Silicon, vorzugsweise ein Silicon enthalten, das ausgewählt ist unter:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter: substituierten oder unsubstituierten aminierten Gruppen; (per)fluorierten Gruppen; Thiolgruppen; Carboxylatgruppen; hydroxylierten Gruppen; alkoxylierten Gruppen; Acyloxyalkylgruppen; amphoteren Gruppen; Bisulfitgruppen; Hydroxyacylaminogruppen; Carboxygruppen; Sulfonsäuregruppen; und Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropften Siliconpolymeren mit Polysiloxan-Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;
oder deren Gemischen.

Erfindungsgemäß besonders bevorzugte Mittel enthalten das bzw. die Silikon(e) vorzugsweise in Mengen von 0,1 bis 10 Gew.-%, vorzugsweise von 0,25 bis 7 Gew.-% und insbesondere von 0,5 bis 5 Gew.-%, jeweils bezogen auf das gesamte Mittel.

Erfindungsgemäß geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Silikonöle bewirken die unterschiedlichsten Effekte. So beeinflussen sie beispielsweise gleichzeitig die Trocken- und Nasskämmbarkeiten, den Griff des trockenen und nassen Haares sowie den Glanz. Unter dem Begriff Silikonöle versteht der Fachmann mehrere Strukturen Silicium-organischer Verbindungen. Zunächst werden hierunter die Dimethiconole (S1) verstanden. Diese können sowohl linear als auch verzweigt als auch cyclisch oder cyclisch und verzweigt sein. Lineare Dimethiconole können durch die folgende Strukturformel (S1 - I) dargestellt werden:

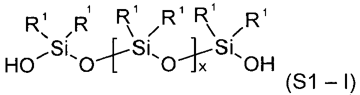

Verzweigte Dimethiconole können durch die Strukturformel (S1 - II) dargestellt werden:

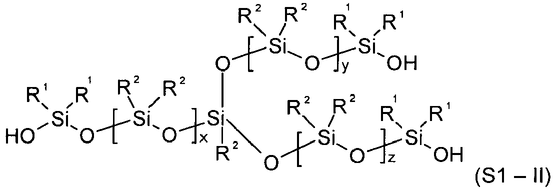

Die Reste R¹ und R² stehen unabhängig voneinander jeweils für Wasserstoff, einen Methylrest, einen C₂ bis C₃₀ linearen, gesättigten oder ungesättigten Kohlenwasserstoffrest, einen Phenylrest und/oder einen Arylrest. Nicht einschränkende Beispiele der durch R¹ und R² repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3-Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R¹ und R² ein Alkylrest, der 1 bis etwa 6 Kohlenstoffatomen enthält, insbesondere bevorzugt ist R¹ und R² Methyl. Die Zahlen x, y und z sind ganze Zahlen und laufen jeweils unabhängig voneinander von 0 bis 50.000. Die Molgewichte der Dimethiconole liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, ganz besonders bevorzugte Viskositäten liegen zwischen 10000 und 3000000 cPs. Der bevorzugteste Bereich liegt zwischen 50000 und 2000000 cPs.

Als Beispiele für derartige Produkte werden die folgenden Handelsprodukte genannt: Botanisil NU-150M (Botanigenics), Dow Corning 1-1254 Fluid, Dow Corning 2-9023 Fluid, Dow Corning 2-9026 Fluid, Ultrapure Dimethiconol (Ultra Chemical), Unisil SF-R (Universal Preserve), X-21-5619 (Shin-Etsu Chemical Co.), Abil OSW 5 (Degussa Care Specialties), ACC DL-9430 Emulsion (Taylor Chemical Company), AEC Dimethiconol & Sodium Dodecylbenzenesulfonate (A & E Connock (Perfumery & Cosmetics) Ltd.), B C Dimethiconol Emulsion 95 (Basildon Chemical Company, Ltd.), Cosmetic Fluid 1401, Cosmetic Fluid 1403, Cosmetic Fluid 1501, Cosmetic Fluid 1401DC (alle zuvor genannten Chemsil Silicones, Inc.), Dow Corning 1401 Fluid, Dow Corning 1403 Fluid, Dow Corning 1501 Fluid, Dow Corning 1784 HVF Emulsion, Dow Corning 9546 Silicone Elastomer Blend (alle zuvor genannten Dow Corning Corporation), Dub Gel SI 1400 (Stearinerie Dubois Fils), HVM 4852 Emulsion (Crompton Corporation), Jeesilc 6056 (Jeen International Corporation), Lubrasil, Lubrasil DS (beide Guardian Laboratories), Nonychosine E, Nonychosine V (beide Exsymol), SanSurf Petrolatum-25, Satin Finish (beide Collaborative Laboratories, Inc.), Silatex-D30 (Cosmetic Ingredient Resources), Silsoft 148, Silsoft E-50, Silsoft E-623 (alle zuvor genannten Crompton Corporation), SM555, SM2725, SM2765, SM2785 (alle zuvor genannten GE Silicones), Taylor T-Sil CD-1, Taylor TME-4050E (alle Taylor Chemical Company), TH V 148 (Crompton Corporation), Tixogel CYD-1429 (Sud-Chemie Performance Additives), Wacker-Belsil CM 1000, Wacker-Belsil CM 3092, Wacker-Belsil CM 5040, Wacker-Belsil DM 3096, Wacker-Belsil DM 3112 VP, Wacker-Belsil DM 8005 VP, Wacker-Belsil DM 60081 VP (alle zuvor genannten Wacker-Chemie GmbH).

Dimethicone (S2) bilden die zweite Gruppe der Silikone, welche erfindungsgemäß enthalten sein können. Diese können sowohl linear als auch verzweigt als auch cyclisch oder cyclisch und verzweigt sein. Lineare Dimethicone können durch die folgende Strukturformel (S2 - I) dargestellt werden:

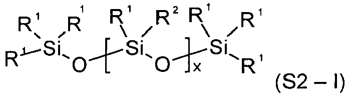

Verzweigte Dimethicone können durch die Strukturformel (S2 - II) dargestellt werden:

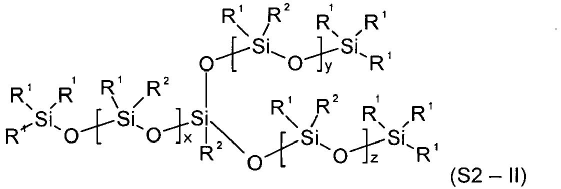

Die Reste R¹ und R² stehen unabhängig voneinander jeweils für Wasserstoff, einen Methylrest, einen C₂ bis C₃₀ linearen, gesättigten oder ungesättigten Kohlenwasserstoffrest, einen Phenylrest und/oder einen Arylrest. Nicht einschränkende Beispiele der durch R¹ und R² repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3-Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R¹ und R² ein Alkylrest, der 1 bis etwa 6 Kohlenstoffatomen enthält, und besonders bevorzugt ist R¹ und R² Methyl. Die Zahlen x, y und z sind ganze Zahlen und laufen jeweils unabhängig voneinander von 0 bis 50.000. Die Molgewichte der Dimethicone liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, besonders bevorzugte Viskositäten liegen zwischen 10000 und 3000000 cPs. Ganz besonders bevorzugt liegt die Viskosität im Bereich zwischen 50000 und 2000000 cPs.

Dimethiconcopolyole (S3) bilden eine weitere Gruppe von Silikonen, die geeignet sind. Dimethiconcopolyole können durch die folgenden Strukturformeln dargestellt werden:

(SiR¹₃)-O-(SiR²₂-O-)ₓ-(SiR²PE-O-)_{y}-(SiR¹₃) (S3 - I),

PE-(SiR¹₂)-O-(SiR²₂-O-)ₓ-(SiR¹₂)-PE (S3 - II) Verzweigte Dimethiconcopolyole können durch die Strukturformel (S3 - III) dargestellt werden:

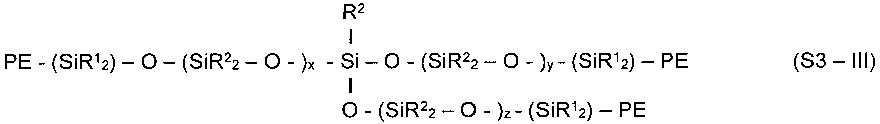

oder durch die Strukturformel (S3 - IV):

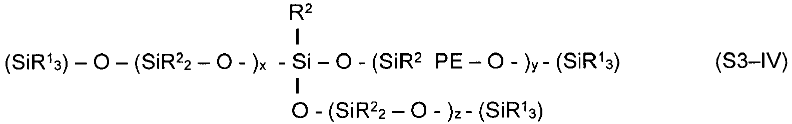

Die Reste R¹ und R² stehen unabhängig voneinander jeweils für Wasserstoff, einen Methylrest, einen C₂ bis C₃₀ linearen, gesättigten oder ungesättigten Kohlenwasserstoffrest, einen Phenylrest und/oder einen Arylrest. Nicht einschränkende Beispiele der durch R¹ und R² repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3-Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R¹ und R² ein Alkylrest, der 1 bis etwa 6 Kohlenstoffatomen enthält, insbesondere bevorzugt ist R¹ und R² Methyl. PE steht für einen Polyoxyalkylenrest. Bevorzugte Polyoxyalkylenreste leiten sich ab von Ethylenoxid, Propylenoxid und Glycerin. Die Zahlen x, y und z sind ganze Zahlen und laufen jeweils unabhängig voneinander von 0 bis 50.000. Die Molgewichte der Dimethicone liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, ganz besonders bevorzugte Viskositäten liegen zwischen 10000 und 3000000 cPs. Der bevorzugteste Bereich liegt zwischen 50000 und 2000000 cPs. Entsprechende Dimethiconcopolyole sind kommerziell erhältlich und werden beispielsweise von der Firma Dow Corning unter der Bezeichnung Dow Corning ® 5330 Fluid vertrieben.

Selbstverständlich umfasst die erfindungsgemäße Lehre auch, dass die Dimethiconole, Dimethicone und/oder Dimethiconcopolymere bereits als Emulsion vorliegen können. Dabei kann die entsprechende Emulsion der Dimethiconole, Dimethicone und/oder Dimethiconcopolyole sowohl nach der Herstellung der entsprechenden Dimethiconole, Dimethicone und/oder Dimethiconcopolyole aus diesen und den dem Fachmann bekannten üblichen Verfahren zur Emulgierung hergestellt werden. Hierzu können als Hilfsmittel zur Herstellung der entsprechenden Emulsionen sowohl kationische, anionische, nichtionische oder zwitterionische Tenside und Emulgatoren als Hilfsstoffe verwendet werden. Selbstverständlich können die Emulsionen der Dimethiconole, Dimethicone und/oder Dimethiconcopolyole auch direkt durch ein Emulsionspolymerisationsverfahren hergestellt werden. Auch derartige Verfahren sind dem Fachmann wohl bekannt.

Wenn die Dimethiconole, Dimethicone und/oder Dimethiconcopolyole als Emulsion verwendet werden, dann beträgt die Tröpfchengröße der emulgierten Teilchen erfindungsgemäß 0,01 bis 10000 µm, bevorzugt 0,01 bis 100 µm, besonders bevorzugt 0,01 bis 20 µm und ganz besonders bevorzugt 0,01 bis 10 µm. Die Teilchengröße wird dabei nach der Methode der Lichtstreuung bestimmt.

Werden verzweigte Dimethiconole, Dimethicone und/oder Dimethiconcopolyole verwendet, so ist darunter zu verstehen, dass die Verzweigung größer ist, als eine zufällige Verzweigung, welche durch Verunreinigungen der jeweiligen Monomere zufällig entsteht. Im Sinne der vorliegenden Erfindung ist daher unter verzweigten Dimethiconolen, Dimethiconen und/oder Dimethiconcopolyolen zu verstehen, dass der Verzweigungsgrad größer als 0,01 % ist. Bevorzugt ist ein Verzweigungsgrad größer als 0,1 % und ganz besonders bevorzugt von größer als 0,5 %. Der Grad der Verzweigung wird dabei aus dem Verhältnis der unverzweigten Monomeren zu den verzweigenden Monomeren, das heißt der Menge an tri- und tetrafunktionalen Siloxanen, bestimmt. Erfindungsgemäß können sowohl niedrigverzweigte als auch hochverzweigte Dimethiconole, Dimethicone und/oder Dimethiconcopolyole ganz besonders bevorzugt sein.

Geeignete Silikone sind weiterhin aminofunktionelle Silikone (S4), insbesondere die Silikone, die unter der INCI-Bezeichnung Amodimethicone zusammengefasst sind. Darunter sind Silikone zu verstehen, welche mindestens eine, gegebenenfalls substituierte, Aminogruppe aufweisen.
Solche Silikone können z.B. durch die Formel (S4 - I)

M(RₐQ_{b}SiO_{(4-a-b)/2)})ₓ(R_{c}SiO_{(4-c)/2)})_{y}M (S4 - I)

beschrieben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel -R¹Z ist, worin R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silikon-Endgruppe ist, wie sie im Stand der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3-Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von R¹ schließen Methylen, Ethylen, Propylen, Hexamethylen, Decamethylen, -CH₂CH(CH₃)CH₂-, Phenylen, Naphthylen, -CH₂CH₂SCH₂CH₂-, CH₂CH₂OCH₂-, -OCH₂CH₂-, -OCH₂CH₂CH₂-, -CH₂CH(CH₃)C(O)OCH₂-, -(CH₂)₃C(O)OCH₂CH₂-,-C₆H₄C₆H₄-, -C₆H₄CH₂C₆H₄-; und -(CH₂)₃C(O)SCH₂CH₂- ein.

Z ist ein organischer, aminofunktioneller Rest, enthaltend mindestens eine funktionelle Aminogruppe. Eine mögliche Formel für Z ist NH(CH₂)_{z}NH₂, worin z für eine ganze Zahl von 1 bis 50 steht. Eine andere mögliche Formel für Z ist -NH(CH₂)_{z}NH(CH₂)_{zz}, worin sowohl z als auch zz unabhängig voneinander für eine ganze Zahl von 1 bis 50 stehen, wobei diese Struktur Diamino-Ringstrukturen umfasst, wie Piperazinyl. Z ist insbesondere bevorzugt ein -NHCH₂CH₂NH₂-Rest. Eine andere mögliche Formel für Z ist - N(CH₂)_{z}NX¹X² oder -NX¹X², worin X¹ und X² jeweils unabhängig voneinander ausgewählt ist aus Wasserstoff und einem Kohlenwasserstoffrest mit 1 bis etwa 6 Kohlenstoffatomen. Ganz besonders bevorzugt steht Q für einen polaren, aminfunktionellen Rest der Formel -CH₂CH₂CH₂NHCH₂CH₂NH₂.

Das molare Verhältnis der RₐQ_{b} SiO_{(4-a-b)/2}-Einheiten zu den R_{c}SiO _{(4-c)/2}-Einheiten liegt im Bereich von etwa 1 : 2 bis 1 : 65, vorzugsweise von etwa 1 : 5 bis etwa 1 : 65 und besonders bevorzugt von etwa 1 : 15 bis etwa 1 : 20. Werden ein oder mehrere Silikone der obigen Formel eingesetzt, dann können die verschiedenen variablen Substituenten in der obigen Formel bei den verschiedenen Silikonkomponenten, die in der Silikonmischung vorhanden sind, verschieden sein.
Bevorzugte aminofunktionelle Silikone entsprechen der Formel (S4 - II)

R'ₐG₃₋ₐ-Si(OSiG₂)ₙ-(OSiG_{b}R'_{2-b})ₘ-O-SiG₃₋ₐ-R'ₐ (S4 - II),

worin bedeutet:
- G ist -H, eine Phenylgruppe, -OH, -O-CH₃, -CH₃, -CH₂CH₃, -CH₂CH₂CH₃, -CH(CH₃)₂, -CH₂CH₂CH₂CH₃, -CH₂CH(CH₃)₂, -CH(CH₃)CH₂CH₃, -C(CH₃)₃ ;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus -N(R")-CH₂-CH₂- N(R")₂, -N(R")₂,-N⁺(R")₃A⁻,
   -N⁺H(R")₂A⁻, -N⁺H₂(R")A⁻, -N(R")-CH₂-CH₂-N⁺R"H₂A⁻,
      wobei jedes R" für gleiche oder verschiedene Reste aus der Gruppe -H,-Phenyl, -Benzyl, der C₁₋₂₀-Alkylreste, vorzugsweise -CH₃, -CH₂CH₃, -CH₂CH₂CH₃, -CH(CH₃)₂, -CH₂CH₂CH₂CH₃, -CH₂CH(CH₃)₂, -CH(CH₃)CH₂CH₃, -C(CH₃)₃, steht und A⁻ ein Anion repräsentiert, welches vorzugsweise ausgewählt ist aus Chlorid, Bromid, lodid oder Methosulfat.

Besonders bevorzugte aminofunktionelle Silikone entsprechen der Formel (S4 - III) 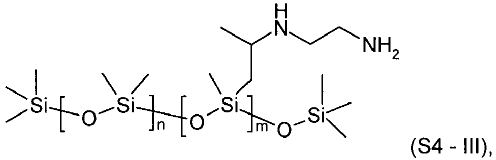 worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Trimethylsilylamodimethicone bezeichnet.
Besonders bevorzugt sind weiterhin aminofunktionelle Silikone der Formel (S4 - IV) 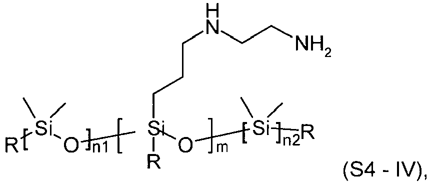 worin R für -OH, -O-CH₃ oder eine -CH₃-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Amodimethicone bezeichnet und sind beispielsweise in Form einer Emulsion als Handelsprodukt Dow Corning® 949 im Gemisch mit einem kationischen und eine nichtionischen Tensid erhältlich.

Vorzugsweise werden solche aminofunktionellen Silikone eingesetzt, die eine Aminzahl oberhalb von 0,25 meq/g, vorzugsweise oberhalb von 0,3 meq/g und insbesondere bevorzugt oberhalb von 0,4 meq/g aufweisen. Die Aminzahl steht dabei für die Milli-Äquivalente Amin pro Gramm des aminofunktionellen Silikons. Sie kann durch Titration ermittelt und auch in der Einheit mg KOH/g angegeben werden.
Weitere geeignete Silikone sind beispielsweise
- oligomere Polydimethylcyclosiloxane (INCI-Bezeichnung: Cyclomethicone), insbesondere die tetramere und die pentamere Verbindung, die als Handelsprodukte DC 245 Fluid, DC 344 bzw. DC 345 von Dow Corning vertrieben werden,
- Hexamethyl-Disiloxan (INCI-Bezeichnung: Hexamethyldisiloxane), z. B. das unter der Bezeichnung Abil® K 520 vertriebenen Produkt,
- Polyphenylmethylsiloxane (INCI-Bezeichnung: Phenyl Trimethicone), z. B. das Handelsprodukt DC 556 Cosmetic Grade Fluid von Dow Corning,
- Ester sowie Partialester der Silikon-Glykol-Copolymere, wie sie beispielsweise von der Firma Fanning unter der Handelsbezeichnung Fancorsil® LIM (INCI-Bezeichnung: Dimethicone Copolyol Meadowfoamate) vertrieben werden,
- anionische Silikonöle, wie beispielsweise das Produkt Dow Corning®1784.

Gemäß einer bevorzugten Ausführungsform enthält das erfindungsgemäße Mittel mindestens zwei unterschiedliche Silikonderivate, insbesondere bevorzugt eine Kombination aus einem flüchtigen und einem nichtflüchtigen Silikon. Flüchtig im Sinne der Erfindung sind solche Silikone, die eine Flüchtigkeit aufweisen, die gleich oder größer als die Flüchtigkeit des cyclischen, pentameren Dimethylsiloxans ist. Solche Kombinationen sind auch als Handelsprodukte (z. B. Dow Corning®1401, Dow Corning®1403 und Dow Corning®1501, jeweils Mischungen aus einem Cyclomethicone und einem Dimethiconol) erhältlich.

Bevorzugte Mischungen verschiedener Silikone sind beispielsweise Dimethicone und Dimethiconole, lineare Dimethicone und cylische Dimethiconole. Eine ganz besonders bevorzugte Mischung von Silikonen besteht aus mindestens einem cyclischen Dimethiconol und/oder Dimethicon, mindestens einem weiteren nicht cylischen Dimethicon und/oder Dimethiconol sowie mindestens einem aminofunktionellen Silikon.

Werden unterschiedliche Silikone als Mischung verwendet, so ist das Mischungsverhältnis weitgehend variabel. Bevorzugt werden jedoch alle zur Mischung verwendeten Silikone in einem Verhältnis von 5:1 bis 1:5 im Falle einer binären Mischung eingesetzt. Ein Verhältnis von 3:1 bis 1:3 ist besonders bevorzugt. Ganz besonders bevorzugte Mischungen enthalten alle in der Mischung enthaltenen Silikone weitestgehend in einem Verhältnis von etwa 1:1, jeweils bezogen auf die eingesetzten Mengen in Gew.-%.

Besonders bevorzugte Silikone werden nachstehend beschrieben. Besonders bevorzugte erfindungsgemäße Mittel sind dadurch gekennzeichnet, daß sie mindestens ein Silikon der Formel Si-I

(CH₃)₃Si-[O-Si(CH₃)₂]ₓ-O-Si(CH₃)₃ (Si-I),

enthalten, in der x für eine Zahl von 0 bis 100, vorzugsweise von 0 bis 50, weiter bevorzugt von 0 bis 20 und insbesondere 0 bis 10, steht. Diese Silikone werden nach der INCI-Nomenklatur als DIMETHICONE bezeichnet. Bevorzugt eingesetzt werden (CH₃)₃Si--O-Si(CH₃)₃ und/oder (CH₃)₃Si-O-(CH₃)₂Si-O-Si(CH₃)₃ und/oder (CH₃)₃Si-[O-(CH₃)₂Si]₂-O-Si(CH₃)₃ besonders bevorzugt sind. Selbstverständlich können auch Mischungen der o.g. Silikone in den erfindungsgemäßen Mitteln enthalten sein.

Bevorzugte erfindungsgemäß einsetzbare Silikone weisen bei 20°C Viskositäten von 0,2 bis 2 mm²s⁻¹ auf, wobei Silikone mit Viskositäten von 0,5 bis 1 mm²s⁻¹ besonders bevorzugt sind.

Besonders bevorzugte erfindungsgemäße Mittel enthalten ein oder mehrere aminofunktionelle Silicone. Solche Silicone können z.B. durch die Formel

M(RₐQ_{b}SiO_{(4-a-b)/2)x}(R_{c}SiO_{(4-c)/2)y}M

beschrieben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel -R¹HZ ist, worin R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silicon-Endgruppe ist, wie sie im Stande der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von R¹ schließen Methylen, Ethylen, Propylen, Hexamethylen, Decamethylen, -CH₂CH(CH₃)CH₂-, Phenylen, Naphthylen, -CH₂CH₂SCH₂CH₂-,-CH₂CH₂OCH₂-, -OCH₂CH₂-, -OCH₂ CH₂CH₂-, -CH₂CH(CH₃)C(O)OCH₂-, -(CH₂)₃CC(O)OCH₂CH₂-, -C₆H₄C₆H₄-, -C₆H₄CH₂C₆H₄-; und -(CH₂)₃C(O)SCH₂CH₂- ein.

Z ist ein organischer, aminofunktioneller Rest, enthaltend mindestens eine funktionelle Aminogruppe. Eine mögliche Formel für Z ist NH(CH₂)_{z}NH₂, worin z 1 oder mehr ist. Eine andere mögliche Formel für Z ist -NH(CH₂)_{z}(CH₂)_{zz}NH, worin sowohl z als auch zz unabhängig 1 oder mehr sind, wobei diese Struktur Diamino-Ringstrukturen umfaßt, wie Piperazinyl. Z ist am bevorzugtesten ein -NHCH₂CH₂NH₂-Rest. Eine andere mögliche Formel für Z ist - N(CH₂)_{z}(CH₂)_{zz}NX₂ oder -NX₂, worin jedes X von X₂ unabhängig ausgewählt ist aus der Gruppe bestehend aus Wasserstoff und Alkylgruppen mit 1 bis 12 Kohlenstoffatomen, und zz 0 ist.

Q ist am bevorzugtesten ein polarer, aminfunktioneller Rest der Formel-CH₂CH₂CH₂NHCH₂CH₂NH₂. In den Formeln nimmt "a" Werte im Bereich von etwa 0 bis etwa 2 an, "b" nimmt Werte im Bereich von etwa 2 bis etwa 3 an, "a" + "b" ist kleiner als oder gleich 3, und "c" ist eine Zahl im Bereich von etwa 1 bis etwa 3. Das molare Verhältnis der RₐQ_{b} SiO_{(4-a-b)/2}-Einheiten zu den R_{c}SiO _{(4-c)/2}-Einheiten liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silicone der obigen Formel eingesetzt, dann können die verschiedenen variablen Substituenten in der obigen Formel bei den verschiedenen Siliconkomponenten, die in der Siliconmischung vorhanden sind, verschieden sein.

Bevorzugte erfindungsgemäße Mittel sind dadurch gekennzeichnet, daß sie ein aminofunktionelles Silikon der Formel (Si-II)

R'ₐG₃₋ₐ-Si(OSiG₂)ₙ-(OSiG_{b}R'_{2-b})ₘ-O-SiG₃₋ₐ-R'ₐ (Si-II),

enthalten, worin bedeutet:
- G ist-H, eine Phenylgruppe, -OH, -O-CH₃, -CH₃, -O-CH₂CH₃, -CH₂CH₃, -O-CH₂CH₂CH₃,-CH₂CH₂CH₃, -O-CH(CH₃)₂, -CH(CH₃)₂, -O-CH₂CH₂CH₂CH₃, -CH₂CH₂CH₂CH₃, -O-CH₂CH(CH₃)₂, -CH₂CH(CH₃)₂, -O-CH(CH₃)CH₂CH₃, -CH(CH₃)CH₂CH₃, -O-C(CH₃)₃,-C(CH₃)₃ ;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus -Q-N(R")-CH₂-CH₂-N(R")₂, -Q-N(R")₂, -Q-N⁺(R")₃A⁻, -Q-N⁺H(R")₂A⁻, -Q-N⁺H₂(R")A⁻, -Q-N(R")-CH₂-CH₂-N⁺R"H₂A-,
   wobei jedes Q für eine chemische Bindung, -CH₂-, -CH₂-CH₂-, -CH₂CH₂CH₂-, -C(CH₃)₂-, -CH₂CH₂CH₂CH₂-, -CH₂C(CH₃)₂-, -CH(CH₃)CH₂CH₂- steht,
   R" für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃,-CH(CH₃)₂, -CH₂CH₂CH₂H₃, -CH₂CH(CH₃)₂, -CH(CH₃)CH₂CH₃, -C(CH₃)₃, steht und A ein Anion repräsentiert, welches vorzugsweise ausgewählt ist aus Chlorid, Bromid, lodid oder Methosulfat.

Besonders bevorzugte erfindungsgemäße Mittel sind dadurch gekennzeichnet, daß sie mindestens ein aminofunktionelles Silikon der Formel (Si-IIa) 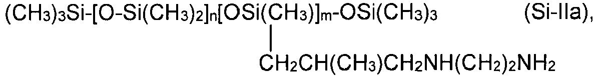 enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt. Diese Silicone werden nach der INCI-Deklaration als Trimethylsilylamodimethicone bezeichnet.

Besonders bevorzugt sind auch erfindungsgemäße Mittel, die ein aminofunktionelles Silikon der Formel (Si-Ilb) 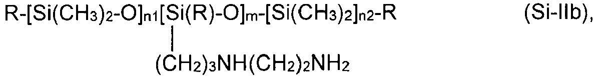 enthalten, worin R für -OH, -O-CH₃ oder eine -CH₃-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt. Diese Silicone werden nach der INCI-Deklaration als Amodimethicone bezeichnet.

Unabhängig davon, welche aminofunktionellen Silicone eingesetzt werden, sind erfindungsgemäße Mittel bevorzugt, die ein aminofunktionelles Silikon enthalten dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silicons. Sie kann durch Titration ermittelt und auch in der Einheit mg KOH/g angegeben werden.

Erfindungsgemäß bevorzugte Mittel sind dadurch gekennzeichnet, dass sie, bezogen auf ihr Gewicht, 0,01 bis 10 Gew.%, vorzugsweise 0,1 bis 8 Gew.%, besonders bevorzugt 0,25 bis 7,5 Gew.% und insbesondere 0,5 bis 5 Gew.% aminofunktionelle(s) Silikon(e) enthalten.

Auch die nach INCI als CYCLOMETHICONE bezeichneten cyclischen Dimethicone sind erfindungsgemäß mit Vorzug einsetzbar. Hier sind erfindungsgemäße Mittel bevorzugt, die mindestens ein Silikon der Formel Si-III 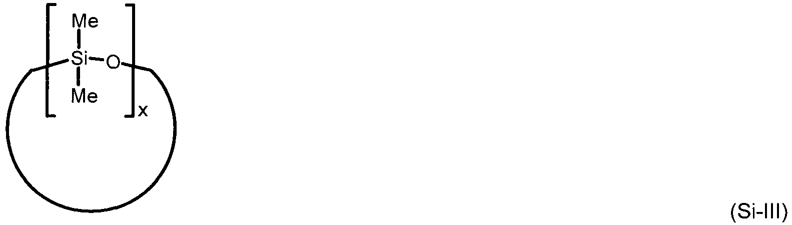 enthalten, in der x für eine Zahl von 3 bis 200, vorzugsweise von 3 bis 10, weiter bevorzugt von30 bis 7 und insbesondere 3, 4, 5 oder 6, steht.

Die vorstehend beschriebenen Silicone weisen ein Rückgrat auf, welches aus -Si-O-Si-Einheiten aufgebaut ist. Selbstverständlich können diese Si-O-Si-Einheiten auch durch Kohlenstoffketten unterbrochen sein. Entsprechende Moleküle sind durch Kettenverlängerungsreaktionen zugänglich und kommen vorzugsweise in Form von Silikon-in-Wasser-Emulsionen zum Einsatz.

Erfindungsgemäß ebenfalls bevorzugte Mittel sind dadurch gekennzeichnet, daß sie mindestens ein Silikon der Formel Si-IV

R₃Si-[O-SiR₂]ₓ-(CH₂)ₙ-[O-SiR₂]_{y}-O-SiR₃ (Si-IV),

enthalten, in der R für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, -CH(CH₃)₂, -CH₂CH₂CH₂H₃, -CH₂CH(CH₃)₂, -CH(CH₃)CH₂CH₃, -C(CH₃)₃, steht, x bzw. y für eine Zahl von 0 bis 200, vorzugsweise von 0 bis 10, weiter bevorzugt von 0 bis 7 und insbesondere 0, 1, 2, 3, 4, 5 oder 6, stehen, und n für eine Zahl von 0 bis 10, bevorzugt von 1 bis 8 und insbesondere für 2, 3, 4, 5, 6 steht.

Die erfindungsgemäßen Mittel enthalten als weiteren Inhaltsstoff vorzugsweise ein filmbildendes Polymer. Besonders bevorzugte erfindungsgemäße Mittel enthalten bezogen auf ihr Gewicht 0,1 bis 25 Gew.-% mindestens eines Filmbildners. Besonders bevorzugte Filmbildner, die in den erfindungsgemäßen Mitteln eingesetzt werden können, werden nachstehend beschrieben.

Besonders bevorzugte erfindungsgemäße Mittel sind dadurch gekennzeichnet, daß sie mindestens ein weiteres Copolymer B enthalten, das mindestens eine Struktureinheit gemäß Formel (B-I) und mindestens eine Struktureinheit der Formel (B-II) enthält, 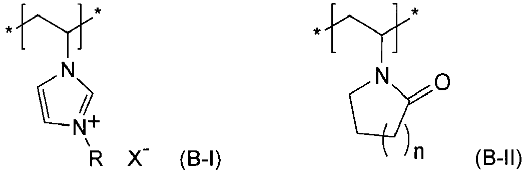 worin
- R eine C₁- bis C₃₀-Alkylgruppe, eine C₁- bis C₄-Aralkylgruppe, eine C₂- bis C₆-Alkenylgruppe oder eine C₂ bis C₆-Hydroxyalkylgruppe bedeutet und
- X⁻ für ein physiologisch verträgliches Anion steht
- n für 1, 2 oder 3 als Anzahl der Methyleneinheiten steht.

Filmbildende und/oder festigende Copolymere B sind bekannt. Diese Copolymere besitzen mindestens eine Struktureinheit gemäß Formel (B-I) und mindestens eine Struktureinheit gemäß Formel (B-II) und können darüber hinaus weitere Struktureinheiten aufweisen, die durch das Hinzufügen entsprechender Monomere bei der Polymerisation einpolymerisiert werden.

Bevorzugte Gruppen R sind beispielsweise -CH₃; -CH₂CH₃, -CH₂CH₂CH₃, CH(CH₃)₂,-(CH₂)₃CH₃,
-CH₂-CH(CH₃)₂, CH(CH₃)CH₂CH₃, -C(CH₃)₃, -CH₂OH, -CH₂CH₂OH, -CH₂CH₂CH₂OH,-CH(OH)CH₂CH₃,
-CH₂CH(OH)CH₃ X⁻ steht für ein physiologisch verträgliches Anion, bevorzugte Anionen sind Chlorid, Bromid, lodid, Sulfat, Methosulfat, Ethylsulfat, Tosylat und Tetrafluoroborat.
In Formel (B-II) steht n für die Anzahl der Methylengruppen. Mit n = 1 steht Formel (B-II) für eine Vinylpyrrolidon-Einheit, mit n = 2 für eine Vinylpiperidinon-Einheit und mit n = 3 für eine Vinylcaprolactam-Einheit. Besonders bevorzugte erfindungsgemäße Mittel sind dadurch gekennzeichnet, daß sie als Copolymer B ein Copolymer B1 enthalten, das mindestens eine Struktureinheit gemäß Formel (B-I) und mindestens eine Struktureinheit der Formel (B-II) enthält, 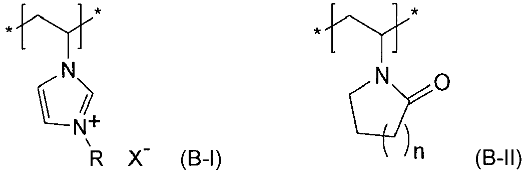 worin R für eine Methylgruppe, X⁻ für Methosulfat und n für 1 Methyleneinheiten steht.

Ganz besonders bevorzugte Copolymere B1 enthalten 10 bis 30 Mol-%, vorzugsweise 15 bis 25 Mol.-% und insbesondere 20 Mol.-% Struktureinheiten gemäß Formel (B-I) und 70 bis 90 Mol.-%, vorzugsweise 75 bis 85 Mol.-% und insbesondere 80 Mol.-% Struktureinheiten gemäß Formel (B-II).
Hierbei ist besonders bevorzugt, wenn die Copolymere B1 neben Polymereinheiten, die aus dem Einbau der genannten Struktureinheiten gemäß Formel (B-I) und (B-II) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere B1 ausschließlich aus Struktureinheiten der Formel (B-I) und (B-II) aufgebaut und lassen sich durch die allgemeine Formel 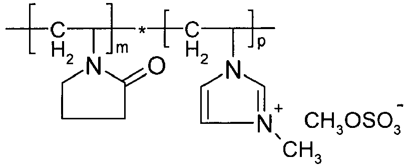 beschreiben, wobei die Indices m und n je nach Molmasse des Polymers variieren und nicht bedeuten sollen, daß es sich zwingend um Blockcopolymere handelt. Vielmehr können Struktureinheiten der Formel (B-I) und der Formel (B-II) im Molekül statistisch verteilt vorliegen. Solche N-Methylvinylimidazol/Vinylpyrrolidon-Copolymere werden laut INCI-Nomenklatur als POLYQUATERNIUM-44 bezeichnet und sind beispielsweise von der BASF unter dem Handelsnamen Luviquat® UltraCare erhältlich. Besonders bevorzugte erfindungsgemäße Mittel enthalten ein Copolymer B1, das Molmassen innerhalb eines bestimmten Bereiches aufweist. Hier sind erfindungsgemäße Mittel bevorzugt, bei denen das Copolymer B1 eine Molmasse von 50 bis 400 kDa, vorzugsweise von 100 bis 300 kDa, weiter bevorzugt von 150 bis 250 kDa und insbesondere von 190 bis 210 kDa aufweist.

Zusätzlich zu dem bzw. den Copolymer(en) B1 oder an dessen bzw. deren Stelle können die erfindungsgemäßen Mittel auch Copolymere B2 enthalten, die als zusätzliche Struktureinheiten Struktureinheiten der Formel (B-II) aufweisen, in der n für die Zahl 3 steht. Weitere besonders bevorzugte erfindungsgemäße Mittel sind dadurch gekennzeichnet, daß sie als Copolymer B ein Copolymer B2 enthalten, das das mindestens eine Struktureinheit gemäß Formel (B-I) und mindestens eine Struktureinheit der Formel (B-II) mit n=1 und mindestens eine weitere Struktureinheit der Formel (B-II) mit n=3 enthält, 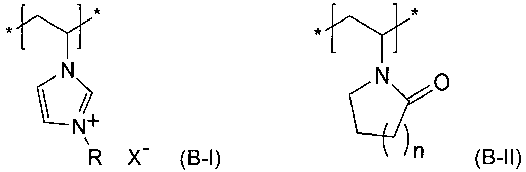 worin R für eine Methylgruppe, X⁻ für Methosulfat steht. Auch hierbei ist besonders bevorzugt, wenn die Copolymere B2 neben Polymereinheiten, die aus dem Einbau der genannten Struktureinheiten gemäß Formel (B-I) und (B-II) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere B2 ausschließlich aus Struktureinheiten der Formel (B-I) und (B-II) aufgebaut und lassen sich durch die allgemeine Formel 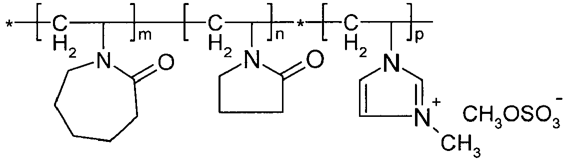 beschreiben, wobei die Indices m, n und p je nach Molmasse des Polymers variieren und nicht bedeuten sollen, daß es sich zwingend um Blockcopolymere handelt. Vielmehr können Struktureinheiten der Formel (B-I) und der Formel (B-II) im Molekül statistisch verteilt vorliegen. Solche N-Methylvinylimidazol/Vinylpyrrolidon/Vinylcaprolactam-Copolymere werden laut INCI-Nomenklatur als POLYQUATERNIUM-46 bezeichnet und sind beispielsweise von der BASF unter dem Handelsnamen Luviquat® Hold erhältlich.

Ganz besonders bevorzugte Copolymere B2 enthalten 1 bis 20 Mol-%, vorzugsweise 5 bis 15 Mol.-% und insbesondere 10 Mol.-% Struktureinheiten gemäß Formel (B-I) und 30 bis 50 Mol.-%, vorzugsweise 35 bis 45 Mol.-% und insbesondere 40 Mol.-% Struktureinheiten gemäß Formel (B-II) mit n = 1 und 40 bis 60 Mol.-%, vorzugsweise 45 bis 55 Mol.-% und insbesondere 60 Mol.-% Struktureinheiten gemäß Formel (B-II) mit n = 3. Besonders bevorzugte erfindungsgemäße Mittel enthalten ein Copolymer B2, das Molmassen innerhalb eines bestimmten Bereiches aufweist. Hier sind erfindungsgemäße Mittel bevorzugt, bei denen das Copolymer B2 eine Molmasse von 100 bis 1000 kDa, vorzugsweise von 250 bis 900 kDa, weiter bevorzugt von 500 bis 850 kDa und insbesondere von 650 bis 710 kDa aufweist.

Zusätzlich zu dem bzw. den Copolymer(en) B1 und/oder B2 oder an dessen bzw. deren Stelle können die erfindungsgemäßen Mittel auch Copolymere B3 enthalten, die als zusätzliche Struktureinheiten Struktureinheiten der Formel (B-II) aufweisen, in der n für die Zahl 3 steht sowie weitere Distruktureinheiten aus der Gruppe der Vinylimidazol-Einheiten und weitere Struktureinheiten aus der Gruppe der Acrylamid- und/oder Methacrylamid-Einheiten. Weitere besonders bevorzugte erfindungsgemäße Mittel sind dadurch gekennzeichnet, daß sie als Copolymer B ein Copolymer B3 enthalten, das mindestens eine Struktureinheit gemäß Formel (B-I) und mindestens eine Struktureinheit der Formel (B-II) und mindestens eine Struktureinheit der Formel (B-III) und mindestens eine Struktureinheit der Formel (B-IV) enthält, 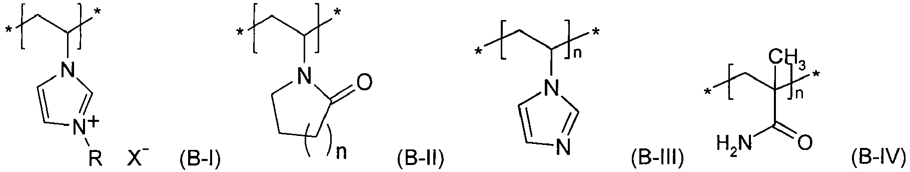 worin R für eine Methylgruppe, X für Methosulfat und n für 1 Methyleneinheiten steht. Auch hierbei ist besonders bevorzugt, wenn die Copolymere B3 neben Polymereinheiten, die aus dem Einbau der genannten Struktureinheiten gemäß Formel (B-I), (B-II), (B-III) und (B-IV) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere B3 ausschließlich aus Struktureinheiten der Formel (B-I), (B-II), (B-III) und (B-IV) aufgebaut und lassen sich durch die allgemeine Formel 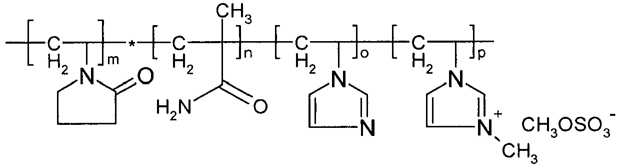 beschreiben, wobei die Indices m, n, o und p je nach Molmasse des Polymers variieren und nicht bedeuten sollen, daß es sich zwingend um Blockcopolymere handelt. Vielmehr können Struktureinheiten der Formeln (B-I), (B-II), (B-III) und (B-IV) im Molekül statistisch verteilt vorliegen.

Solche N-Methylvinylimidazol/Vinylpyrrolidon/Vinylimidazol/Methacrylamid-Copolymere werden laut INCI-Nomenklatur als POLYQUATERNIUM-68 bezeichnet und sind beispielsweise von der BASF unter dem Handelsnamen Luviquat® Supreme erhältlich.

Ganz besonders bevorzugte Copolymere B3 enthalten 1 bis 12 Mol-%, vorzugsweise 3 bis 9 Mol.-% und insbesondere 6 Mol.-% Struktureinheiten gemäß Formel (B-I) und 45 bis 65 Mol.-%, vorzugsweise 50 bis 60 Mol.-% und insbesondere 55 Mol.-% Struktureinheiten gemäß Formel (B-II) mit n = 1 und 1 bis 20 Mol.-%, vorzugsweise 5 bis 15 Mol.-% und insbesondere 10 Mol.-% Struktureinheiten gemäß Formel (B-III) und 20 bis 40 Mol.-%, vorzugsweise 25 bis 35 Mol.-% und insbesondere 29 Mol.-% Struktureinheiten gemäß Formel (B-IV). Besonders bevorzugte erfindungsgemäße Mittel enthalten ein Copolymer B3, das Molmassen innerhalb eines bestimmten Bereiches aufweist. Hier sind erfindungsgemäße Mittel bevorzugt, bei denen das Copolymer B3 eine Molmasse von 100 bis 500 kDa, vorzugsweise von 150 bis 400 kDa, weiter bevorzugt von 250 bis 350 kDa und insbesondere von 290 bis 310 kDa aufweist.

Unabhängig davon, ob nur ein Copolymer B oder mehrere Copolymere C eingesetzt werden und unabhängig von der Wahl des speziellen Copolymers B sind erfindungsgemäße Mittel bevorzugt, bei denen die Gesamtmenge an Copolymeren B, bezogen auf das Gewicht des anwendungsbereiten Mittels, 0,05 bis 5 Gew.-%, vorzugsweise 0,1 bis 4 Gew.-% und insbesondere 0,25 bis 3 Gew.-%, beträgt.

Unabhängig davon, ob nur ein Copolymer B oder mehrere Copolymere B eingesetzt werden und unabhängig von der Wahl des speziellen Copolymers B sind erfindungsgemäße Mittel bevorzugt, bei denen die Gesamtmenge an Copolymeren B, bezogen auf das Gewicht des anwendungsbereiten Mittels, 0,05 bis 5 Gew.-%, vorzugsweise 0,1 bis 4 Gew.-% und insbesondere 0,25 bis 3 Gew.-%, beträgt.

Zusätzlich zu den filmbildenden Copolymeren B oder an deren Stelle können die erfindungsgemäßen Mittel weitere filmbildende Polymere C aus der Gruppe der Acrylat-Polymere enthalten, d.h. der Polymere, die mindestens eine Monomereinheit aus der Gruppe Acrylsäure und/oder Methacrylsäure und/oder deren Estern enthalten. Bevorzugte erfindungsgemäße Mittel enthalten mindestens ein Acrylat-Polymer C, ausgewählt aus c1) Polyacrylsäure und/oder c2) Copolymeren von Methacrylsäure mit Acrylamidopropansulfonsäure und/oder c3) Copolymeren von Acrylsäure mit Methacrylsäure und Acrylsäureestern und/oder c4) Copolymeren von Acrylsäure mit Methacrylsäure mit Acrylsäureestern und Methacrylsäureestern und/oder c5) Copolymeren von Acrylsäureestern mit Methacrylsäure.

So sind beispielsweise erfindungsgemäße Mittel bevorzugt, die als Polymer C Polyacrylsäure enthalten. Diese weist Struktureinheiten der Formel 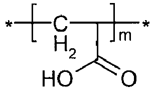 auf, in der m je nach Molmasse variiert.

Besonders bevorzugte erfindungsgemäße Mittel sind dadurch gekennzeichnet, daß sie als Polymer c1 Polyacrylsäuren mit einer Molmasse von 10 bis 250 kDa, vorzugsweise von 25 bis 200 kDa, weiter bevorzugt von 50 bis 150 kDa und insbesondere von 70 bis 100 kDa, enthalten.

Vorzugsweise werden die Polymere c1 innerhalb bestimmter Mengenbereiche eingesetzt. Hier sind erfindungsgemäße Mittel bevorzugt, die, bezogen auf das Gewicht des anwendungsbereiten Mittels, 0,05 bis 5 Gew.-%, vorzugsweise 0,1 bis 4 Gew.-% und insbesondere 0,25 bis 3 Gew.-% Polymer(e) c1 enthalten.

Zusätzlich zu dem bzw. den Polymer(en) c1 oder an dessen bzw. deren Stelle können die erfindungsgemäßen Mittel auch Polymere c2 aus der Gruppe der Copolymeren von Methacrylsäure mit Acrylamidopropansulfonsäure enthalten. Diese lassen sich durch die allgemeine Formel 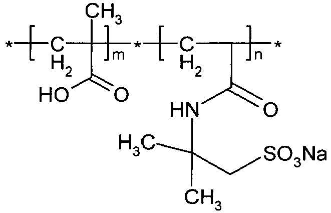 beschreiben, wobei die Indices m und n je nach Molmasse des Polymers variieren und nicht bedeuten sollen, daß es sich zwingend um Blockcopolymere handelt. Vielmehr können Struktureinheiten im Molekül statistisch verteilt vorliegen.

Besonders bevorzugte erfindungsgemäße Mittel sind dadurch gekennzeichnet, daß sie als Copolymer c2 Copolymere von Methacrylsäure mit Acrylamidopropansulfonsäure mit einer Molmasse von 100 bis 2500 kDa, vorzugsweise von 250 bis 2000 kDa, weiter bevorzugt von 500 bis 1750 kDa und insbesondere von 800 bis 1500 kDa, enthalten.

Vorzugsweise werden die Copolymere c2 innerhalb bestimmter Mengenbereiche eingesetzt. Hier sind erfindungsgemäße Mittel bevorzugt, die, bezogen auf das Gewicht des anwendungsbereiten Mittels, 0,05 bis 5 Gew.-%, vorzugsweise 0,1 bis 4 Gew.-% und insbesondere 0,25 bis 3 Gew.-% Copolymer(e) c2 enthalten. Copolymere von Methacrylsäure und Acrylamidopropansulfonsäure sind beispielsweise unter dem Handelsnamen Fixomer® A-30 (Nalco) erhältlich.

Zusätzlich zu dem bzw. den Polymer(en) c1 und/oder den Copolymer(en) c2 oder an dessen bzw. deren Stelle können die erfindungsgemäßen Mittel auch Polymere c3 aus der Gruppe der Copolymeren von Acrylsäure mit Methacrylsäure und Acrylsäureestern enthalten.
Diese lassen sich durch die allgemeine Formel 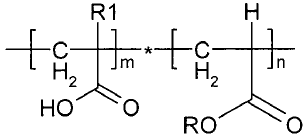 beschreiben, wobei die Indices m und n je nach Molmasse des Polymers variieren und nicht bedeuten sollen, daß es sich zwingend um Blockcopolymere handelt. Vielmehr können Struktureinheiten im Molekül statistisch verteilt vorliegen. R1 steht für-H oder -CH₃.

Besonders bevorzugte erfindungsgemäße Mittel sind dadurch gekennzeichnet, daß sie als Copolymer c3 Copolymere von Acrylsäure mit Methacrylsäure und Acrylsäureestern mit einer Molmasse von 50 bis 500 kDa, vorzugsweise von 100 bis 400 kDa, weiter bevorzugt von 150 bis 300 kDa und insbesondere von 200 bis 250 kDa, enthalten.

Vorzugsweise werden die Copolymere c3 innerhalb bestimmter Mengenbereiche eingesetzt. Hier sind erfindungsgemäße Mittel bevorzugt, die, bezogen auf das Gewicht des anwendungsbereiten Mittels, 0,05 bis 5 Gew.-%, vorzugsweise 0,1 bis 4 Gew.-% und insbesondere 0,25 bis 3 Gew.-% Copolymer(e) c3 enthalten. Ein ganz besonders bevorzugtes Copolymer c3 wird nach der INCI-Nomenklatur als Acrylates Copolymer bezeichnet. Ein solches Polymer ist beispielsweise unter der Handelsbezeichnung Aculyn® 33A (Rohm & Haas) erhältlich.

Zusätzlich zu dem bzw. den Polymer(en) c1 und/oder den Copolymer(en) c2 und/oder den Copolymer(en) c3 oder an dessen bzw. deren Stelle können die erfindungsgemäßen Mittel auch Polymere c4 aus der Gruppe der Copolymere von Acrylsäure mit Methacrylsäure und ethoxylierten Acrylsäureestern und ethoxylierten Methacrylsäureestern enthalten.
Diese lassen sich durch die allgemeine Formel 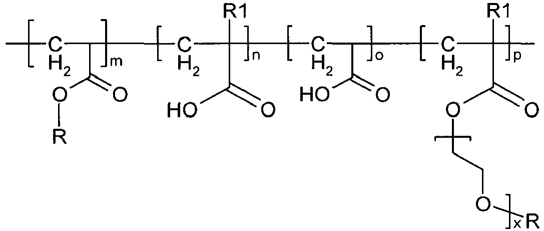 beschreiben, wobei die Indices m, n, o und p je nach Molmasse des Polymers variieren und nicht bedeuten sollen, daß es sich zwingend um Blockcopolymere handelt. Vielmehr können Struktureinheiten im Molekül statistisch verteilt vorliegen. R1 steht für eine Methylgruppe, der Rest R für einen Kohlenwasserstoffrest mit einem bis 22 C-Atomen, x steht für 1 bis 50.

Besonders bevorzugte erfindungsgemäße Mittel sind dadurch gekennzeichnet, daß sie als Copolymer c4 Copolymere von Acrylsäure mit Methacrylsäure und ethoxylierten Acrylsäureestern und ethoxylierten Methacrylsäureestern mit einer Molmasse von 100 bis 500 kDa, vorzugsweise von 150 bis 400 kDa, weiter bevorzugt von 200 bis 300 kDa und insbesondere von 225 bis 275 kDa, enthalten.

Vorzugsweise werden die Copolymere c4 innerhalb bestimmter Mengenbereiche eingesetzt. Hier sind erfindungsgemäße Mittel bevorzugt, die, bezogen auf das Gewicht des anwendungsbereiten Mittels, 0,05 bis 5 Gew.-%, vorzugsweise 0,1 bis 4 Gew.-% und insbesondere 0,25 bis 3 Gew.-% Copolymer(e) c4 enthalten.

Besonders bevorzugte Copolymere c4 weisen 20 bis 30 EO-Einheiten auf (x = 20, 21, 22, 23, 24, 25, 26, 27, 28, 29, 30) und besitzen als Rest R einen Stearylrest oder Behenylrest. Ein ganz besonders bevorzugtes Copolymer c4 besitzt 25 EO-Einheiten, ist mit Behenylalkohol verestert und wird nach der INCI-Nomenklatur als Acrylates/Beheneth-25 Methacrylate Copolymer bezeichnet. Ein solches Polymer ist beispielsweise unter der Handelsbezeichnung Aculyn® 28 (Rohm & Haas) erhältlich.

Zusätzlich zu dem bzw. den Polymer(en) c1 und/oder den Copolymer(en) c2 und/oder den Copolymer(en) c3 und/oder den Copolymer(en) c4 oder an dessen bzw. deren Stelle können die erfindungsgemäßen Mittel auch Polymere c5 aus der Gruppe der Copolymere von Acrylsäureestern mit Methacrylsäure enthalten. Bevorzugte Acrylsäureester sind Methylacrylat und Ethylacrylat, wobei letzteres besonders bevorzugt ist. Besonders bevorzugte erfindungsgemäße Mittel sind dadurch gekennzeichnet, daß sie als Copolymer c5 Copolymere von Acrylsäureestern mit Methacrylsäure mit einer Molmasse von 100 bis 500 kDa, vorzugsweise von 150 bis 400 kDa, weiter bevorzugt von 200 bis 300 kDa und insbesondere von 225 bis 275 kDa, enthalten.

Vorzugsweise werden die Copolymere c5 innerhalb bestimmter Mengenbereiche eingesetzt. Hier sind erfindungsgemäße Mittel bevorzugt, die, bezogen auf das Gewicht des anwendungsbereiten Mittels, 0,05 bis 5 Gew.-%, vorzugsweise 0,1 bis 4 Gew.-% und insbesondere 0,25 bis 3 Gew.-% Copolymer(e) c5 enthalten. Ein ganz besonders bevorzugtes Copolymer c5 entstammt der Polymerisation von Metacrylsäure mit Ethylacrylat und wird nach der INCI-Nomenklatur als Acrylates Copolymer bezeichnet. Ein solches Polymer ist beispielsweise unter der Handelsbezeichnung Luviflex® Soft (BASF) erhältlich.

Ganz besonders bevorzugte erfindungsgemäße Mittel sind daher dadurch gekennzeichnet, daß sie ein Copolymer D aus Methacrylsäure und Ethylacrylat enthalten.

Vorzugsweise werden Copolymere D eingesetzt, die 10 bis 80 Mol-%, vorzugsweise 20 bis 70 Mol.-%, besonders bevorzugt 30 bis 60 Mol.-% und insbesondere 40 bis 50 Mol.-% Methacrylsäure und 20 bis 90 Mol.-%, vorzugsweise 30 bis 80 Mol.-%, besonders bevorzugt 40 bis 70 Mol.-% und insbesondere 50 bis 60 Mol.-% Ethylacrylat enthalten.

Noch weiter bevorzugt sind erfindungsgemäße Mittel, bei denen das Copolymer D eine Molmasse von 100 bis 800 kDa, vorzugsweise von 200 bis 700 kDa, weiter bevorzugt von 300 bis 600 kDa und insbesondere von 450 bis 550 kDa aufweist. Besonders bevorzugt enthält das erfindungsgemäße Mittel das Copolymer, das von der Firma BASF AG unter der Bezeichnung Luviflex® Soft (INCI-Bezeichnung: Acrylates Copolymer) vertrieben wird.

Unabhängig davon, welche(s) Copolymer(e) C bzw. D eingesetzt wird bzw. werden, sind erfindungsgemäße Mittel bevorzugt, bei denen die Gesamtmenge an Copolymeren C bzw. D, bezogen auf das Gewicht des anwendungsbereiten Mittels, 0,05 bis 5 Gew.-%, vorzugsweise 0,1 bis 4 Gew.-% und insbesondere 0,25 bis 3 Gew.-%, beträgt.

Hinsichtlich der Auswahl der zusätzlich einsetzbaren Copolymere B und C bzw. D (D ist ein bevorzugtes Polymer c5) unterliegt die vorliegende Erfindung keinen Beschränkungen. Es können sowohl jeweils nur ein Polymer als auch jeweils mehrere Polymere aus den einzelnen beschriebenen Klassen eingesetzt werden. Besonders bevorzugte Mittel enthalten zusätzlich zu dem erfindungsgemäß zwingend eingesetzten Copolymer A und dem zwingend vorhandenen mindestens einem Silikon

Es sind erfindungsgemäße Mittel bevorzugt, bei denen das Gewichtsverhältnis von Polymer(en) B zu Polymer(en) C 10:1 bis 1:10, vorzugsweise 8:1 bis 1:8, weiter bevorzugt 5:1 bis 1:5 und insbesondere 4:1 bis 1:4 beträgt. Unabhängig von Art und Gewichtsverhältnis der Polymeren zueinander sind darüber hinaus erfindungsgemäße Mittel bevorzugt, bei denen der Gesamt-Polymergehalt (B + C + D) der Mittel 1 bis 15 Gew.-%, vorzugsweise 2,5 bis 12,5 Gew.-%, weiter bevorzugt 4 bis 10 Gew.-% und insbesondere 5 bis 8 Gew.-% beträgt.

Zusätzlich zu den filmbildenden Copolymeren B und/oder C und/oder D oder an deren Stelle können die erfindungsgemäßen Mittel weitere filmbildende Polymere E aus der Gruppe der Acrylat-Polymere enthalten, d.h. der Polymere, die mindestens eine Monomereinheit aus der Gruppe Acrylsäure und/oder Methacrylsäure und/oder deren Estern enthalten. Bevorzugte erfindungsgemäße Mittel enthalten mindestens ein Copolymer E, gebildet aus mindestens einem Monomer e1 ausgewählt aus Acrylsäure und/oder Methacrylsäure, und mindestens einem Monomer e2 ausgewählt aus Acrylamid und/oder Methacrylamid und mindestens einem Monomer e3 ausgewählt aus N-substituierten Acrylamiden und/oder Methacrylamiden.

Dieses Copolymer E beinhaltet mindestens ein Monomer e1 ausgewählt aus Acrylsäure und/oder Methacrylsäure, und mindestens ein Monomer e2 ausgewählt aus Acrylamid und/oder Methacrylamid und mindestens ein Monomer e3 ausgewählt aus N-substituierten Acrylamiden und/oder Methacrylamiden und kann darüber hinaus weitere Struktureinheiten aufweisen, die durch das Hinzufügen entsprechender Monomere bei der Polymerisation einpolymerisiert werden.

Besonders bevorzugte Copolymere A sind Copolymere aus Acrylsäure und Acrylamid und N-substituierten Acrylamiden; Acrylsäure und Methacrylamid und N-substituierten Acrylamiden; Methacrylsäure und Acrylamid und N-substituierten Acrylamiden; Methacrylsäure und Methacrylamid und N-substituierten Acrylamiden und/oder Methacrylamiden; Acrylsäure und Acrylamid und N-substituierten Methacrylamiden; Acrylsäure und Methacrylamid und N-substituierten Methacrylamiden; Methacrylsäure und Acrylamid und N-substituierten Methacrylamiden; Methacrylsäure und Methacrylamid und N-substituierten Methacrylamiden. Besonders bevorzugte erfindungsgemäße Mittel sind dadurch gekennzeichnet, daß sie als Copolymer E ein Copolymer E1 enthalten, welches als Monomer e1 Acrylsäure umfaßt.

Ein bevorzugte Monomer ist das Acrylamid. Demnach sind bevorzugte erfindungsgemäße Mittel dadurch gekennzeichnet, daß sie als Copolymer E ein Copolymer E1 enthalten, welches als Monomer e2 Acrylamid umfaßt.
Die N-Substitution an den N-substituierten Acrylamiden kann durch einfache Alkalgruppen (vorzugsweise Methyl-, Ethyl-, n-Propyl, Isoporpyl) erfolgen, besonders bevorzugt sind jedoch substituierte Alkylgruppen, die anionische Funktionalitäten tragen. Ganz besonders bevorzugt sind Sulfonatgruppenhaltige Substituenten.

Ein erfindungsgemäß besonders bevorzugtes Mittel ist dadurch gekennzeichnet, daß es als Copolymer E ein Copolymer E1 enthält, welches als Monomer e3 Acryloyldimethyltaurat umfaßt. Diese Copolymere E1 lassen sich durch die allgemeine Formel 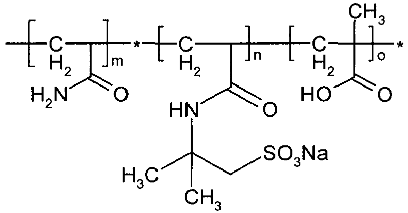 beschreiben, wobei die Indices m, n und o je nach Molmasse des Polymers variieren und nicht bedeuten sollen, daß es sich zwingend um Blockcopolymere handelt. Vielmehr können Struktureinheiten im Molekül statistisch verteilt vorliegen.

Besonders bevorzugte erfindungsgemäße Mittel sind dadurch gekennzeichnet, daß das Copolymer E1 eine Molmasse von 50 bis 500 kDa, vorzugsweise von 100 bis 450 kDa, weiter bevorzugt von 150 bis 400 kDa und insbesondere von 200 bis 300 kDa aufweist.

Vorzugsweise werden die Copolymere E innerhalb bestimmter Mengenbereiche eingesetzt. Hier sind erfindungsgemäße Mittel bevorzugt, bei denen die Gesamtmenge an Copolymeren E, bezogen auf das Gewicht des anwendungsbereiten Mittels, 0,05 bis 5 Gew.-%, vorzugsweise 0,1 bis 4 Gew.-% und insbesondere 0,25 bis 3 Gew.-% beträgt. Copolymere von Acrylamid mit Methacrylsäure und Acryloyldimethyltaurat sind beispielsweise unter dem Handelsnamen Acudyne® SCP (Rohm & Haas) erhältlich.

Zusätzlich zu den filmbildenden Copolymeren B und/oder C und/oder D und/oder E oder an deren Stelle können die erfindungsgemäßen Mittel weitere filmbildende Polymere F enthalten. Bevorzugte erfindungsgemäße Mittel enthalten mindestens ein Copolymer F, ausgewählt aus f1) Copolymeren von Vinylpyrrolidon mit Methacrylamidopropyltrimethylammoniumchlorid (MAPTAC) und/oder f2) Copolymeren von Vinylpyrrolidon mit Dimethylaminoethylmethacrylat und/oder f3) Copolymeren von Vinylpyrrolidon mit Dimethylaminopropylmethacrylamid und Alkyldimethylpropylmethacrylamidoammoniumsalzen. So sind beispielsweise erfindungsgemäße Mittel bevorzugt, die als Polymer F Copolymere von Vinylpyrrolidon mit Methacrylamidopropyltrimethylammoniumchlorid (MAPTAC) enthalten (b1).
Diese lassen sich durch die allgemeine Formel 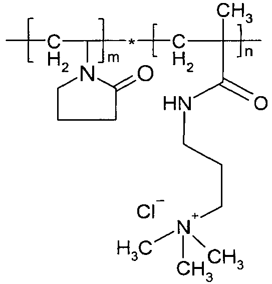 beschreiben, wobei die Indices m und n je nach Molmasse des Polymers variieren und nicht bedeuten sollen, daß es sich zwingend um Blockcopolymere handelt. Vielmehr können Struktureinheiten im Molekül statistisch verteilt vorliegen.

Besonders bevorzugte erfindungsgemäße Mittel sind dadurch gekennzeichnet, daß sie als kationisches Polymer f1 Copolymere von Methacrylamidopropyltrimethylammoniumchlorid (MAPTAC) mit Vinylpyrrolidon enthalten, die 40 bis 95 Mol.-%, vorzugsweise 42,5 bis 90 Mol.-%, weiter bevorzugt 45 bis 85 Mol.-% und insbesondere 50 bis 80 Mol.-% Vinylpyrrolidon enthalten.

Besonders bevorzugte erfindungsgemäße Mittel sind weiter dadurch gekennzeichnet, daß die Copolymere f1 Molmassen von 10 bis 1000 kDa, vorzugsweise von 25 bis 900 kDa, weiter bevorzugt von 50 bis 800 kDa und insbesondere von 100 bis 750 kDa, aufweisen.

Vorzugsweise werden die Copolymere f1 innerhalb bestimmter Mengenbereiche eingesetzt. Hier sind erfindungsgemäße Mittel bevorzugt, die, bezogen auf das Gewicht des anwendungsbereiten Mittels, 0,05 bis 5 Gew.-%, vorzugsweise 0,1 bis 4 Gew.-% und insbesondere 0,25 bis 3 Gew.-% Copolymer(e) f1 enthalten. Ein ganz besonders bevorzugtes Copolymer f1 wird nach der INCI-Nomenklatur als Polyquaternium-28 bezeichnet. Ein solches Polymer ist beispielsweise unter der Handelsbezeichnung Gafquat® HS-100 (ISP) erhältlich.

Zusätzlich zu dem bzw. den Polymer(en) f1 oder an dessen bzw. deren Stelle können die erfindungsgemäßen Mittel auch Polymere f2 aus der Gruppe der Copolymeren von Vinylpyrrolidon mit Dimethylaminoethylmethacrylat enthalten.
Diese lassen sich durch die allgemeine Formel 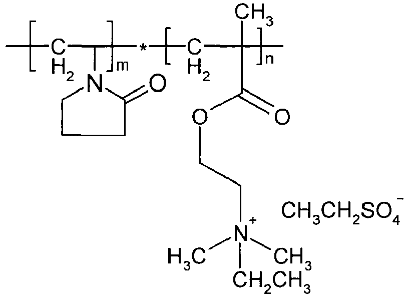 beschreiben, wobei die Indices m und n je nach Molmasse des Polymers variieren und nicht bedeuten sollen, daß es sich zwingend um Blockcopolymere handelt. Vielmehr können Struktureinheiten im Molekül statistisch verteilt vorliegen.

Besonders bevorzugte erfindungsgemäße Mittel sind dadurch gekennzeichnet, daß sie als kationisches Polymer f2 Copolymere von Vinylpyrrolidon mit Dimethylaminoethylmethacrylat enthält, die 40 bis 95 Mol.-%, vorzugsweise 42,5 bis 90 Mol.-%, weiter bevorzugt 45 bis 85 Mol.-% und insbesondere 50 bis 80 Mol.-% Vinylpyrrolidon enthalten.

Besonders bevorzugte erfindungsgemäße Mittel sind weiter dadurch gekennzeichnet, daß die Copolymere f2 Molmassen von 100 bis 2500 kDa, vorzugsweise von 250 bis 2000 kDa, weiter bevorzugt von 500 bis 1750 kDa und insbesondere von 800 bis 1500 kDa, aufweisen.

Vorzugsweise werden die Copolymere f2 innerhalb bestimmter Mengenbereiche eingesetzt. Hier sind erfindungsgemäße Mittel bevorzugt, die, bezogen auf das Gewicht des anwendungsbereiten Mittels, 0,05 bis 5 Gew.-%, vorzugsweise 0,1 bis 4 Gew.-% und insbesondere 0,25 bis 3 Gew.-% Copolymer(e) f2 enthalten. Ein ganz besonders bevorzugtes Copolymer f2 wird nach der INCI-Nomenklatur als Polyquaternium-11 bezeichnet. Ein solches Polymer ist beispielsweise unter der Handelsbezeichnung Gafquat® 755 N (ISP) erhältlich.

Zusätzlich zu dem bzw. den Polymer(en) f1 und/oder den Polymer(en) ff2 oder an dessen bzw. deren Stelle können die erfindungsgemäßen Mittel auch Polymere f3 aus der Gruppe der Copolymeren von Vinylpyrrolidon mit Dimethylaminopropylmethacrylamid und Alkyldimethylpropylmethacrylamidoammoniumsalzen enthalten.
Diese lassen sich durch die allgemeine Formel 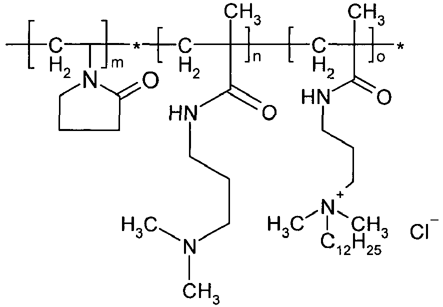 beschreiben, wobei die Indices m, n und o je nach Molmasse des Polymers variieren und nicht bedeuten sollen, daß es sich zwingend um Blockcopolymere handelt. Vielmehr können Struktureinheiten im Molekül statistisch verteilt vorliegen.

Besonders bevorzugte erfindungsgemäße Mittel sind dadurch gekennzeichnet, daß sie als kationisches Polymer f3 Copolymere von Vinylpyrrolidon mit Dimethylaminopropylmethacrylamid und Lauryldimethylpropylmethacrylamidoammoniumsalzen enthalten.

Besonders bevorzugte erfindungsgemäße Mittel sind weiter dadurch gekennzeichnet, daß sie als kationisches Polymer f3 Copolymere von Vinylpyrrolidon mit Dimethylaminopropylmethacrylamid und Alkyldimethylpropylmethacrylamidoammoniumsalzen enthalten, die 40 bis 95 Mol.-%, vorzugsweise 42,5 bis 90 Mol.-%, weiter bevorzugt 45 bis 85 Mol.-% und insbesondere 50 bis 80 Mol.-% Vinylpyrrolidon enthalten. Ganz besonders bevorzugte erfindungsgemäße Mittel sind darüber hinaus dadurch gekennzeichnet, daß die Copolymere f3 Molmassen von 10 bis 1000 kDa, vorzugsweise von 25 bis 900 kDa, weiter bevorzugt von 50 bis 800 kDa und insbesondere von 100 bis 750 kDa, aufweisen. Vorzugsweise werden die Copolymere f3 innerhalb bestimmter Mengenbereiche eingesetzt. Hier sind erfindungsgemäße Mittel bevorzugt, die, bezogen auf das Gewicht des anwendungsbereiten Mittels, 0,05 bis 5 Gew.-%, vorzugsweise 0,1 bis 4 Gew.-% und insbesondere 0,25 bis 3 Gew.-% Copolymer(e) f3 enthalten. Ein ganz besonders bevorzugtes Copolymer f3 wird nach der INCI-Nomenklatur als Polyquaternium-55 bezeichnet. Ein solches Polymer ist beispielsweise unter der Handelsbezeichnung Styleze® W20 (ISP) erhältlich.

Unabhängig von Art und Gewichtsverhältnis aller in den erfindungsgemäßen Mitteln enthaltenen Polymeren zueinander sind darüber hinaus erfindungsgemäße Mittel bevorzugt, bei denen der Gesamt-Polymergehalt der Mittel 1 bis 15 Gew.-%, vorzugsweise 2,5 bis 12,5 Gew.-%, weiter bevorzugt 4 bis 10 Gew.-% und insbesondere 5 bis 8 Gew.-% beträgt.
Die erfindungsgemäßen Mittel enthalten die Inhaltsstoffe in einem kosmetisch akzeptablen Träger.

Bevorzugte kosmetisch akzeptable Träger sind wässrige, alkoholische oder wässrigalkoholische Medien mit vorzugsweise mindestens 10 Gew.-% Wasser, bezogen auf das gesamte Mittel. Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein.

Als zusätzliche Co-Solventien können organische Lösungsmittel oder ein Gemisch aus Lösungsmitteln mit einem Siedepunkt unter 400°C in einer Menge von 0,1 bis 15 Gewichtsprozent, bevorzugt von 1 bis 10 Gewichtsprozent bezogen auf das gesamte Mittel enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel sind Glycerin, Ethylenglykol, butylenglycol und Propylenglykol in einer Menge bis 30 Gew.-% bezogen auf das gesamte Mittel.

Die Mittel weisen bevorzugt einen pH-Wert von 2 bis 11 auf. Besonders bevorzugt ist der pH-Bereich zwischen 4 und 9. Die Angaben zum pH-Wert beziehen sich dabei im Sinne dieser Schrift auf den pH-Wert bei 25°C, sofern nichts anderes vermerkt ist.

Die erfindungsgemäßen Mittel können weiterhin die Hilfs- und Zusatzstoffe enthalten, die üblicherweise den jeweiligen kosmetischen Mitteln zugesetzt werden.

Als geeignete Hilfs- und Zusatzstoffe sind insbesondere Pflegestoffe zu nennen. Diese finden sowohl bei Haut- als auch Haarbehandlungsmitteln Anwendung und können bei geeigneter Wahl des Pflegestoffs beispielsweise in Cremes, Shampoos, Haarspülungen, Haarkuren, Gele, Pump- und Aerosolsprays und Schaumprodukte eingearbeitet werden.

Als Pflegestoff kann ein erfindungsgemäßes Mittel beispielsweise mindestens ein Proteinhydrolysat und/oder eines seiner Derivate enthalten.
Proteinhydrolysate sind Produktgemische, die durch sauer, basisch oder enzymatisch katalysierten Abbau von Proteinen (Eiweißen) erhalten werden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Erfindungsgemäß können Proteinhydrolysate sowohl pflanzlichen als auch tierischen oder marinen oder synthetischen Ursprungs eingesetzt werden. Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan® (Cognis), Promois® (Interorgana), Collapuron® (Cognis), Nutrilan® (Cognis), Gelita-Sol® (Deutsche Gelatine Fabriken Stoess & Co), Lexein® (Inolex), Sericin (Pentapharm) und Kerasol® (Croda) vertrieben. Besonders interessant ist der Einsatz von Seiden-Proteinhydrolysaten. Die Proteinhydrolysate sind in den erfindungsgemäßen Mitteln beispielsweise in Konzentrationen von 0,01 Gew.-% bis zu 20 Gew.-%, vorzugsweise von 0,05 Gew.-% bis zu 15 Gew.-% und ganz besonders bevorzugt in Mengen von 0,05 Gew.-% bis zu 5 Gew.-%, jeweils bezogen auf die gesamte Anwendungszubereitung enthalten.

Als Pflegestoff einer anderen Verbindungsklasse sind weiterhin kationische Tenside geeignet. Erfindungsgemäß bevorzugt sind kationische Tenside vom Typ der quartären Ammoniumverbindungen, der Esterquats und der Amidoamine. Bevorzugte quartäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z.B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75, Dehyquart® C-4046, Dehyquart® L80 und Dehyquart® AU-35 sind Beispiele für solche Esterquats. Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyl-dimethylamin dar.

Die kationischen Tenside sind in den erfindungsgemäßen Mitteln bevorzugt in Mengen von 0,05 bis 10 Gew.-%, bezogen auf die gesamte Anwendungszubereitung, enthalten. Mengen von 0,1 bis 5 Gew.-% sind besonders bevorzugt.

Als Pflegestoff eignen sich ebenfalls pflegende Polymere. Es sei an dieser Stelle darauf hingewiesen, dass einige pflegende Polymere auch filmbildende und/oder festigende Eigenschaften aufweisen, und daher auch bei der Aufzählung geeigneter filmbildender und/oder festigender Polymere genannt sein können.

Eine erste Gruppe der pflegenden Polymere sind die kationischen Polymere. Unter kationischen Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen.
Homopolymere der allgemeinen Formel (G1-I), 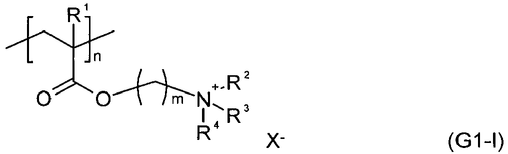 in der R¹= -H oder -CH₃ ist, R², R³ und R⁴ unabhängig voneinander ausgewählt sind aus C₁₋₄-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (G1-I) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt: R¹ steht für eine Methylgruppe, R², R³ und R⁴ stehen für Methylgruppen, m hat den Wert 2.

Als physiologisch verträgliche Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwässrigen Polymerdispersion, die einen Polymeranteil nicht unter 30 Gew.-% aufweisen sollte, eingesetzt. Solche Polymerdispersionen sind unter den Bezeichnungen Salcare® SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare® SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (G1-I) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare® SC 92 erhältlich.
Weitere bevorzugte kationische Polymere sind beispielsweise
- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate,
- kationische Alkylpolyglycoside gemäß der DE-PS 44 13 686,
- kationisierter Honig, beispielsweise das Handelsprodukt Honeyquat® 50,
- kationische Guar-Derivate, wie insbesondere die unter den Handelsnamen Cosmedia®Guar und Jaguar® vertriebenen Produkte,
- Polysiloxane mit quartären Gruppen, wie beispielsweise die im Handel erhältlichen Produkte Q2-7224 (Hersteller: Dow Corning; ein stabilisiertes Trimethylsilylamodimethicon), Dow Corning® 929 Emulsion (enthaltend ein hydroxyl-amino-modifiziertes Silikon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt), diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Weitere erfindungsgemäß einsetzbare kationische Polymere sind die so genannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen® CMF, Hydagen® HCMF, Kytamer® PC und Chitolam® NB/101 im Handel frei verfügbar sind.

Die erfindungsgemäßen Mittel enthalten die pflegenden, kationischen und/oder amphoteren Polymere in bevorzugter Weise in einer Menge von 0,01 bis 5 Gew.-%, insbesondere in einer Menge von 0,1 bis 2 Gew.-%, jeweils bezogen auf die gesamte Anwendungszubereitung.

Weiter bevorzugte erfindungsgemäße Mittel sind dadurch gekennzeichnet, daß sie zusätzlich Pflegestoff(e) - bezogen auf ihr Gewicht - in Mengen von 0,001 bis 10 Gew.-%, vorzugsweise 0,005 bis 7,5 Gew.-%, besonders bevorzugt 0,01 bis 5 Gew.-% und insbesondere 0,05 bis 2,5 Gew.-% enthalten, wobei bevorzugte Pflegstoff(e) ausgewählt sind aus der Gruppe L-Carnitin und/oder seiner Salze; Panthenol und/oder Panthothensäure; der 2-Furanone und/oder deren Derivate (insbesondere Pantolacton); Taurin und/oder seiner Salze; Niacinamid; Ubichinon; Ectoin; Allantoin.

L-Carnitin (IUPAC-Name(*R*)-(3-Carboxy-2-hydroxypropyl)- *N*,*N*,*N-*trimethylammoniumhydroxid), ist eine natürlich vorkommende, vitaminähnliche Substanz. Erfindungsgemäß bevorzugte L-Carnitinderivate sind insbesondere ausgewählt aus Acetyl-L-Carnitin, L-Carnitin-Fumarat, L-Carnitin-Citrat, Lauroyl-L-Carnitin und besonderes bevorzugt L-Carnitin-Tartrat. Die genannten L-Carnitin-Verbindungen sind beispielsweise von der Firma Lonza GmbH (Wuppertal, Deutschland) erhältlich. Erfindungsgemäße bevorzugte Mittel sind dadurch gekennzeichnet, daß sie - bezogen auf ihr Gewicht -0,001 bis 10 Gew.-%, vorzugsweise 0,005 bis 7,5 Gew.-%, besonders bevorzugt 0,01 bis 5 Gew.-% und insbesondere 0,05 bis 2,5 Gew.-% L-Carnitin oder L-Carnitinderivate enthalten, wobei bevorzugte L-Carnitinderivate ausgewählt sind aus Acetyl-L-Carnitin, L-Carnitin-Fumarat, L-Carnitin-Citrat, Lauroyl- L-Carnitin und insbesondere L-Carnitin-Tartrat.

Panthenol (IUPAC-Name: (+)-(R)-2,4-Dihydroxy-N-(3-hydroxypropyl)-3,3-dimethylbutyramid) wird im Körper zu Pantothensäure umgewandelt. Pantothensäure ist ein Vitamin aus der Gruppe der B-Vitamine (Vitamin B5). Erfindungsgemäße bevorzugte Mittel sind dadurch gekennzeichnet, daß sie - bezogen auf sein Gewicht -0,01 bis 5 Gew.-%, vorzugsweise 0,05 bis 2,5 Gew.-%, besonders bevorzugt 0,1 bis 1,5 Gew.-% und insbesondere 0,25 bis 1 Gew.-% Panthenol ((±)-2,4-Dihydroxy-N-(3-hydroxypropyl)-3,3-dimethyl-butyramid) enthalten.

Ein weiterer, bevorzugter einsetzbarer Pflege-Enhancer, der aktivierende Eigenschaften besitzt, ist das Taurin. Erfindungsgemäß bevorzugte Mittel enthalten - bezogen auf ihr Gewicht - 0,01 bis 15 Gew.-%, vorzugsweise 0,025 bis 12,5 Gew.-%, besonders bevorzugt 0,05 bis 10 Gew.-%, weiter bevorzugt 0,1 bis 7,5 Gew.-% und insbesondere 0,5 bis 5 Gew.-% Taurin (2-Aminoethansulfonsäure).

Eine weitere bevorzugte Gruppe von Pflege-Enhancerm in den erfindungsgemäßen Mitteln sind Vitamine, Provitamine oder Vitaminvorstufen. Diese werden nachfolgend beschrieben:
Zusammenfassend sind erfindungsgemäße Mittel bevorzugt, die - bezogen auf ihr Gewicht - 0,1 bis 5 Gew.-%, vorzugsweise 0,2 bis 4 Gew.-%, besonders bevorzugt 0,25 bis 3,5 Gew.-%, weiter bevorzugt 0,5 bis 3 Gew.-% und insbesondere 0,5 bis 2,5 Gew.-% Vitamine und/oder ProVitamine und/oder Vitaminvorstufen enthalten, die vorzugsweise den Gruppen A, B, C, E, F und H zugeordnet werden, wobei bevorzugte Mittel -2,4-Dihydroxy-*N*-(3-hydroxypropyl)-3,3-dimethyl-butyramid, Provitamin B₅) und/oder Pantothensäure (Vitamin B₃, Vitamin B₅) und/oder Niacin, Niacinamid bzw. Nicotinamid (Vitamin B₃) und/oder L-Ascorbinsäure (Vitamin C) und/oder Thiamin (Vitamin B₁) und/oder Riboflavin (Vitamin B₂, Vitamin G) und/oder Biotin (Vitamin B₇, Vitamin H) und/oder Folsäure (Vitamin B₉, Vitamin B_{c} oder Vitamin M) und/oder Vitamin B₆ und/oder Vitamin B₁₂ enthalten.

Es hat sich gezeigt, daß bestimmte Chinone eine besondere Eignung als Pflege-Enhancer besitzen. Besonders bevorzugte erfindungsgemäße Mittel sind dadurch gekennzeichnet, daß sie als Pflegestoff - bezogen auf ihr Gewicht - 0,0001 bis 1 Gew.-%, bevorzugt 0,001 bis 0,5 Gew.-% und besonders bevorzugt 0,005 bis 0,1 Gew.-% mindestens eines Ubichinons und/oder mindestens eines Ubichinols und/oder mindestens eines Derivates dieser Substanzen enthalten, wobei bevorzugte Mittel ein Ubichinon der Formel (Ubi) enthalten 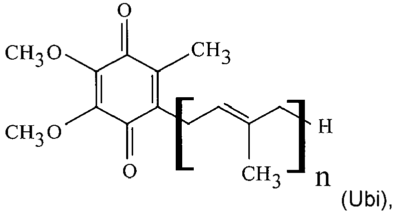 in der n für die Werte = 6, 7, 8, 9 oder 10, besonders bevorzugt für 10 (Coenzym Q10) steht.

Als weiteren Pflege-Enhacer können die erfindungsgemäßen Mittel Ectoin enthalten. Ectoin ((4S)-2-Methyl-1,4,5,6-Tetrahydropyrimidin-4-Carbonsäure) ist ein zur Gruppe der kompatiblen Solute gehörender Naturstoff.

Als weiteren Bestandteil können die erfindungsgemäßen Mittel mindestens ein Kohlenhydrat aus der Gruppe der Monosaccharide, Disaccharide und/oder Oligosaccharide enthalten. Hier sind erfindungsgemäß bevorzugte Haarbehandlungsmittel dadurch gekennzeichnet, daß sie als Pflegestoff - bezogen auf ihr Gewicht - 0,01 bis 5 Gew.-%, vorzugsweise 0,05 bis 4,5 Gew.-%, besonders bevorzugt 0,1 bis 4 Gew.-%, weiter bevorzugt 0,5 bis 3,5 Gew.-% und insbesondere 0,75 bis 2,5 Gew.-% Kohlenhydrat(e), ausgewählt aus Monosacchariden, Disacchariden und/oder Oligosacchariden enthalten, wobei bevorzugte Kohlenhydrate ausgewählt sind aus
- Monosachhariden, insbesondere D-Ribose und/oder D-Xylose und/oder L-Arabinose und/oder D-Glucose und/oder D-Mannose und/oder D-Galactose und/oder D-Fructose und/oder Sorbose und/oder L-Fucose und/oder L-Rhamnose
- Disacchariden, insbesondere Saccharose und/oder Maltose und/oder Lactose und/oder Trehalose und/oder Cellobiose und/oder Gentiobiose und/oder Isomaltose.

In einer weiteren bevorzugten Ausführungsform können die erfindungsgemäßen Mittel Emulgatoren (F) enthalten. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Erfindungsgemäß verwendbare Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov®68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls® PGPH),
- Lineare und verzweigte Fettsäuren mit 8 bis 30 C - Atomen und deren Na-, K-, Ammonium-, Ca-, Mg- und Zn - Salze.
Die erfindungsgemäßen Mittel enthalten die Emulgatoren bevorzugt in Mengen von 0,1 - 25 Gew.-%, insbesondere 0,5 - 15 Gew.-%, bezogen auf das gesamte Mittel. Bevorzugt können die erfindungsgemäßen Zusammensetzungen mindestens einen nichtionogenen Emulgator mit einem HLB-Wert von 8 bis 18 enthalten. Nichtionogene Emulgatoren mit einem HLB-Wert von 10 - 15 können erfindungsgemäß besonders bevorzugt sein.
In Abhängigkeit von der Art des erfindungsgemäßen Mittels kann es erforderlich sein, dass diese weiterhin mindestens ein Tensid enthalten. Dies gilt insbesondere für Hautreinigungsmittel und Shampoos. Aber auch andere Mittel, wie beispielsweise Haarspülungen, Haarkuren und bestimmte Stylingmittel, insbesondere Stylingschäume, können Tenside enthalten.

Beispielsweise können kationische Tenside eingesetzt werden, wie sie bereits oben als geeignete Pflegestoffe beschrieben sind. Bezüglich der bevorzugten kationischen Tenside und der eingesetzten Mengen gelten obige Ausführungen entsprechend.

Neben oder statt der kationischen Tenside können die Mittel weitere Tenside oder Emulgatoren enthalten, wobei prinzipiell sowohl anionische als auch ampholytische und nichtionische Tenside und alle Arten bekannter Emulgatoren geeignet sind. Die Gruppe der ampholytischen oder auch amphoteren Tenside umfasst zwitterionische Tenside und Ampholyte. Die Tenside können bereits emulgierende Wirkung haben.

Als anionische Tenside eignen sich prinzipiell alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate,
- sulfatierte Hydroxyalkylpolyethylen- und/oder Hydroxyalkylenpropylenglykolether,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
   Alkyl- und/oder Alkenyletherphosphate
- sulfatierte Fettsäurealkylenglykolester der Formel (E1-II)

   R⁷CO(AlkO)ₙSO₃M (E1-II)

   in der R⁷CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906 beschrieben sind,
- Amidethercarbonsäuren,
- Kondensationsprodukte aus C₈ - C₃₀ - Fettalkoholen mit Proteinhydrolysaten und/oder Aminosäuren und deren Derivaten, welche dem Fachmann als Eiweissfettsäurekondensate bekannt sind, wie beispielsweise die Lamepon® - Typen, Gluadin® - Typen, Hostapon® KCG oder die Amisoft® - Typen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglycerdisulfate, Alkyl- und Alkenyletherphosphate sowie Eiweissfettsäurekondensate.

Als zwitterionische Tenside werden solche oberflächenaktiven Verbindungen bezeichnet, die im Molekül mindestens eine quartäre Ammoniumgruppe und mindestens eine -COO⁽⁻⁾ - oder-SO₃⁽⁻⁾ -Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter Ampholyten werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete Ampholyte sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte Ampholyte sind das N-Kokosalkylaminopropionat, das Kokosacylaminoethylaminopropionat und das C₁₂ - C₁₈ - Acylsarcosin.

Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol® LS, Dehydol® LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen® HSP (Cognis) oder Sovermol - Typen (Cognis),
- alkoxilierte Triglyceride,
- alkoxilierte Fettsäurealkylester der Formel (E4-I)

   R¹CO-(OCH₂CHR²)_{w}OR³ (E4-I)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R⁴O-[G]ₚ (E4-II)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die bevorzugten Alkyl- und/oder Alkenyloligoglykoside sind somit Alkyl- und/oder Alkenyloligoglucoside. Die Indexzahl p in der allgemeinen Formel (E4-II) gibt den Oligomerisierungsgrad (DP), d. h. die Verteilung von Mono- und Oligoglykosiden an und steht für eine Zahl zwischen 1 und 10. Der Alkyl- bzw. Alkenylrest R⁴ kann sich von primären Alkoholen mit 4 bis 11, vorzugsweise 8 bis 10 Kohlenstoffatomen ableiten. Bevorzugt sind Alkyloligoglucoside auf Basis von gehärtetem C_{12/14}-Kokosalkohol mit einem DP von 1 bis 3.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten. Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Die weiteren Tenside werden in der Regel in Mengen von 0,1 bis 45 Gew.-%, bevorzugt 0,5 bis 30 Gew.-% und ganz besonders bevorzugt von 0,5 bis 25 Gew.-%, bezogen auf die jeweilige gesamte Zusammensetzung, eingesetzt. Dabei hängt die eingesetzte Menge wesentlich davon ab, welchen Zweck das erfindungsgemäße Mittel erfüllt. Handelt es sich um ein Shampoo oder ein anderes reinigendes Mittel, sind auch Tensidmengen über 45 Gew.-% üblich.

Die Formulierung der erfindungsgemäßen Mittel kann in allen für kosmetische Mittel üblichen Formen erfolgen, beispielsweise in Form von Lösungen, die als Gesichts- oder Haarwasser oder Pump- oder Aerosolspray auf die Haut oder das Haar aufgebracht werden können, in Form von Cremes, Emulsionen, Wachsen, Gelen oder auch tensidhaltigen schäumenden Lösungen oder anderen Zubereitungen, die für die Anwendung auf der Haut oder dem Haar geeignet sind.

Vorzugsweise handelt es sich jedoch bei den erfindungsgemäßen Mitteln um Mittel zur temporären Verformung keratinischer Fasern, d.h. um Stylingmittel. Bevorzugte Stylingmittel sind Stylinggele, Pumphaarsprays, Aerosolhaarspray, Pumphaarschäume und Aerosolhaarschäume. Stylinggele ist dabei im Rahmen der vorliegenden Anmeldung der Oberbegriff für klare oder trübe Produkte, Stylingwachse, Stylingcremes, Stylinglotionen, Styling-Jellys usw. Letztlich fallen unter diesen Begriff alle Mittel zum Frisieren von Haaren, die nicht Haarsprays oder Schäume sind.

Unter Haarschäumen werden dabei Zusammensetzungen verstanden, die bei der Entnahme aus einem geeigneten Behälter einen Schaum ausbilden. Es kann notwendig sein, den Mitteln Inhaltsstoffe zuzusetzen, die die Schaumbildung fördern oder einmal gebildeten Schaum stabilisieren. Insbesondere eignen sich dafür Tenside und/oder Emulgatoren, wie sie bereits oben beschrieben wurden. Vorzugsweise werden Tenside aus der Gruppe der kationischen Tenside eingesetzt.

Haarcremes und Haargele enthalten in der Regel Strukturanten und/oder verdickende Polymere, die dazu dienen, den Produkten die gewünschte Konsistenz zu verleihen. Strukturanten und/oder verdickende Polymere werden typischerweise in einer Menge von 0,1 bis 10 Gew.-%, bezogen auf das gesamte Produkt, eingesetzt. Mengen von 0,5 bis 5 Gew.-%, insbesondere 0,5 bis 3 Gew.-% sind bevorzugt. Da die erfindungsgemäß eingesetzte Polymerkombination jedoch selbstverdickende Eigenschaften aufweist, ist die Zugabe weiterer Strukturanten und/oder verdickender Polymere nicht zwingend erforderlich. Vorzugsweise enthalten die erfindungsgemäßen Mittel keine weiteren Strukturanten und/oder verdickender Polymere.

Sofern es sich bei den erfindungsgemäßen Mitteln um ein Aerosolprodukt handelt, enthält dieses zwingend ein Treibmittel. Erfindungsgemäß geeignete Treibmittel sind beispielsweise N₂O, Dimethylether, CO₂, Luft und Alkane mit 3 bis 5 Kohlenstoffatomen, wie Propan, n-Butan, iso-Butan, n-Pentan und iso-Pentan, und deren Mischungen. Bevorzugt sind Dimethylether, Propan, n-Butan, iso-Butan und deren Mischungen. Bevorzugt werden die genannten Alkane, Mischungen der genannten Alkane oder Mischungen der genannten Alkane mit Dimethylether als einziges Treibmittel eingesetzt. Die Erfindung umfasst aber ausdrücklich auch die Mitverwendung von Treibmitteln vom Typ der Fluorchlorkohlenwasserstoffe, insbesondere aber der Fluorkohlenwasserstoffe. Über das Mengenverhältnis von Treibmittel zu den übrigen Bestandteilen der Zubereitungen lassen sich bei gegebener Sprühvorrichtung die Größen der Aerosoltröpfchen bzw. der Schaumblasen und die jeweilige Größenverteilung einstellen.

Die Menge an eingesetztem Treibmittel variiert in Abhängigkeit von der konkreten Zusammensetzung des Mittels, der verwendeten Verpackung und der gewünschten Produktart, etwa Haarspray oder Haarschaum. Bei Verwendung herkömmlicher Sprühvorrichtungen enthalten Aerosolschaumprodukte das Treibmittel bevorzugt in Mengen von 1 bis 35 Gew.-%, bezogen auf das gesamte Produkt. Mengen von 2 bis 30 Gew.-%, insbesondere von 3 bis 15 Gew.-% sind besonders bevorzugt. Aerosolsprays enthalten generell größere Mengen an Treibmittel. Bevorzugt wird das Treibmittel in diesem Fall in einer Menge von 30 bis 98 Gew.-%, bezogen auf das gesamte Produkt, eingesetzt. Mengen von 40 bis 95 Gew.-%, insbesondere von 50 bis 95 Gew.-% sind besonders bevorzugt.

Die Aerosolprodukte lassen sich in üblicher Art und Weise herstellen. In der Regel werden alle Bestandteile des jeweiligen Mittels mit Ausnahme des Treibmittels in einen geeigneten druckfesten Behälter eingefüllt. Dieser wird daraufhin mit einem Ventil verschlossen. Über herkömmliche Techniken wird schließlich die gewünschte Menge Treibmittel eingefüllt.

Ein zweiter Gegenstand der Erfindung ist daher ein Verfahren, bei dem das erfindungsgemäße, kosmetisches Mittel auf das Haar als Pumphaarspray, Aerosolhaarspray, Pumphaarschaum, Aerosolhaarschaum oder Stylinggel aufgetragen wird und gegebenenfalls mit den Handflächen und/oder den Fingern in das Haar eingearbeitet wird.

Für das erfindungsgemäße Verfahren gilt mutatis mutandis das zu den erfindungsgemäßen Mitteln Gesagte. Die gewünschte Verformung der Haare kann dabei mit den Finger oder Händen sowie mit geeigneten, herkömmlichen Hilfsmitteln wie beispielsweise Kamm oder Bürste erfolgen.

Gegenstand der Erfindung ist die Verwendung der erfindungsgemäßen Mittel zur temporären Verformung keratinischer Fasern. Die erfindungsgemäßen Mittel und Produkte, die diese Mittel enthalten, zeichnen sich insbesondere dadurch aus, dass sie behandeltem Haar einen sehr starken Frisurenhalt verleihen, ohne dabei die Haare spröde oder unflexibel zu machen. Vielmehr wird ein angenehmer, weicher Griff erzielt.
Die Formulierung der erfindungsgemäßen Mittel kann in allen für kosmetische Mittel üblichen Formen erfolgen, beispielsweise in Form von Lösungen, die als Gesichts- oder Haarwasser oder Pump- oder Aerosolspray auf die Haut oder das Haar aufgebracht werden können, in Form von Cremes, Emulsionen, Wachsen, Gelen oder auch tensidhaltigen schäumenden Lösungen oder anderen Zubereitungen, die für die Anwendung auf der Haut oder dem Haar geeignet sind.

### Beispiele

Die folgenden Mengenangaben verstehen sich - soweit nichts anderes vermerkt ist - in Gewichtsprozent. Es wurden die erfindungsgemäßen Stylingmittel A bis F gemäß folgender Tabelle hergestellt.

| **Rohstoffe** | **A** | **B** | **C** | **D** | **E** | **F** |
|---|---|---|---|---|---|---|
| Simulgel SMS 88 ¹ | 3,0 | 3,0 | 3,5 | 5,0 | 3,0 | 3,0 |
| Dow Corning 1403 ² | 3,5 | - | - | - | 3,5 | - |
| Silikonöl 50 cSt | 2,0 | 2,0 | - | - | 2,0 | 2,0 |
| Dow Corning 949 ³ | 1,0 | - | - | - | 1,0 | - |
| Dow Corning 5330 ⁴ | 1,5 | 1,5 | - | - | 1,5 | 1,5 |
| Dow Corning 193 C ⁵ | - | - | 0,2 | 0,2 | - | - |
| Abil Quat 3272 ⁶ | - | 1,0 | - | - | - | 1,0 |
| Copolymer aus Vinylpyrrolidon und Vinylacetat, 60%ig in Wasser | 8,0 | 11,0 | 6,0 | 6,5 | 8,0 | 11,0 |
| Luviset Clear ⁷ | - | - | 3,0 | 2,75 | - | - |
| Synthalen K ⁸ | - | - | 1,0 | 1,0 | - | - |
| Phenoxyethanol, Methylisothiazolinone (1:1) | - | 0,5 | 0,5 | 0,5 | 0,5 | 0,5 |
| Benzophenone-4 | 0,05 | 0,05 | 0,05 | 0,05 | 0,05 | 0,05 |
| Glycerin 86%ig | 6, | 6,0 | - | - | 5,0 | 5,0 |
| Dioctylcarbonat | 1,5 | 1,5 | - | - | 1,0 | 1,0 |
| Ethanol 96%ig | 15,0 | - | - | - | - | - |
| D-Panthenol 75%ig | 0,2 | 0,2 | 0,2 | 0,2 | 0,2 | 0,2 |
| Ectoin | - | - | - | - | 0,1 | 0,2 |
| Allantoin | - | - | - | - | 0,1 | 0,1 |
| Coffein | - | - | - | - | 0,1 | 0,1 |
| Parfüm | 0,2 | 0,2 | 0,2 | 0,2 | 0,2 | 0,2 |
| Wasser, vollentsalzt | ad 100 | ad 100 | ad 100 | ad 100 | ad 100 | ad 100 |

| | | | | | | |
|---|---|---|---|---|---|---|
| ¹ Copolymerisat aus Natriumacrylat, Natriumacryloyldimethyltaurat und Dimethylacrylamid (inverse, auto-inversible Latex mit Isohexadecan und Polysorbat-60, ca. 20 Gew.-% Festkörper in Wasser) ² Mischung aus Dimethicon und Dimethiconol (INCI-Bezeichnung: Dimethicone, Dimethiconol) (Dow) ³ Amodimethicon mit Aminzahlen von MEQ-Amine 0.075-0.095/g (ca. 35 Gew.-% Aktivsubstanz in Wasser; INCI-Bezeichnung: AMINODIMETHICONE, CETRIMONIUM CHLORIDE, TRIDECETH-12) (Dow) ⁴ Dimethicon-Copolyol (INCI-Bezeichnung: PEG/PPG-15/15 DIMETHICONE) (Dow) ⁵ Dimethicon-Copolyol (ca. 27 Gew.-% Festkörper in Wasser; INCI-Bezeichnung: PEG-12 DIMETHICONE) (Dow) ⁶ Siloxane und Silicone mit di-Methyl-3-(3-((3-cocoamidopropyl) dimethylammonio)-2-hydroxypropyl)propyl Gruppen, Acetate (49-51%in Propylenglycol; INCI-Bezeichnung: QUATERNIUM-80) (Goldschmidt) ⁷ Copolymerisat Methacrylamid, N-vinylimidazol, N-vinyl-2-pyrrolidinon (ca. 20 Gew.-% Festkörper in Wasser; INCI-Bezeichnung: VP/METHACRYLAMIDE/VINYL IMIDAZOLE COPOLYMER) (BASF) ⁸ Polyacrylsäure (Feststoff); INCI-Bezeichnung: Carbomer (3V Sigma) | | | | | | |

## Patentansprüche

1. Verwendung eines kosmetischen Mittels, enthaltend in einem kosmetisch akzeptablen Träger
a) mindestens ein Copolymer A, das mindestens eine Struktureinheit gemäß Formel (I) und mindestens eine Struktureinheit gemäß Formel (II) und mindestens eine Struktureinheit gemäß Formel (III) enthält 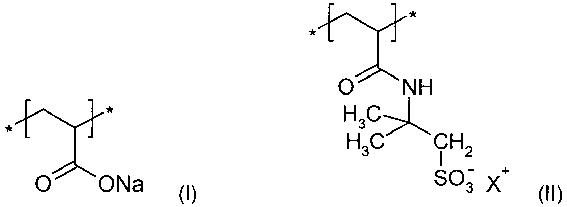 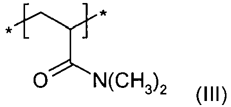 worin X⁺ für ein physiologisch verträgliches Kation steht
und
b) mindestens ein Silikonöl und/oder Silikongum.
zur temporären Verformung keratinischer Fasern.

2. Verwendung nach Anspruch 1, **dadurch gekennzeichnet, dass** es Copolymer(e) A mit Molmassen von 10 bis 750 kDa, vorzugsweise von 25 bis 500 kDa, weiter bevorzugt von 50 bis 400 kDa und insbesondere von 70 bis 250 kDa, enthält.

3. Verwendung nach einem der Ansprüche 1 oder 2, **dadurch gekennzeichnet, dass** es, bezogen auf das Gewicht des anwendungsbereiten Mittels, 0,1 bis 10 Gew.-%, vorzugsweise 0,5 bis 7,5 Gew.-% und insbesondere 1 bis 5 Gew.-% Copolymer(e) A enthält.

4. Verwendung nach einem der Ansprüche 1 bis 3, **dadurch gekennzeichnet, dass** es Copolymer(e) A enthält, die 10 bis 90 Mol.-% (vorzugsweise 15 bis 85 Mol.-% und insbesondere 20 bis 80 Mol.-%) Monomere der Formel (I) und 5 bis 85 Mol.-% (vorzugsweise 7,5 bis 80 Mol.-% und insbesondere 10 bis 60 Mol.-%) Monomere der Formel (II) und 5 bis 85 Mol.-% (vorzugsweise 10 bis 80 Mol.-% und insbesondere 15 bis 70 Mol.-%) Monomere der Formel (III) enthalten.

5. Verwendung nach einem der Ansprüche 1 bis 4, **dadurch gekennzeichnet, dass** mindestens ein Silikon der Formel Si-I
(CH₃)₃Si-[O-Si(CH₃)₂]ₓ-O-Si(CH₃)₃ (Si-I),
enthalten, in der x für eine Zahl von 0 bis 100, vorzugsweise von 0 bis 50, weiter bevorzugt von 0 bis 20 und insbesondere 0 bis 10, steht.

6. Verwendung nach einem der Ansprüche 1 bis 5, **dadurch gekennzeichnet, dass** sie mindestens ein aminofunktionelles Silikon der Formel (Si-II)
R'ₐG₃₋ₐ-Si(OSiG₂)ₙ-(OSiG_{b}R'_{2-b})ₘ-O-SiG₃₋ₐ-R'ₐ (Si-II),
enthalten, worin bedeutet:
- G ist-H, eine Phenylgruppe, -OH, -O-CH₃, -CH₃, -O-CH₂CH₃, -CH₂CH₃, -O-CH₂CH₂CH₃, -CH₂CH₂CH₃, -O-CH(CH₃)₂, -CH(CH₃)₂, -O-CH₂CH₂CH₂CH₃, -CH₂CH₂CH₂CH₃, -O-CH₂CH(CH₃)₂, -CH₂CH(CH₃)₂, -O-CH(CH₃)CH₂CH₃, -CH(CH₃)CH₂CH₃, -O-C(CH₃)₃, -C(CH₃)₃ ;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus -Q-N(R")-CH₂-CH₂-N(R")₂ , -Q-N(R")₂, -Q-N⁺(R")₃A⁻, -Q-N⁺H(R")₂A⁻, -Q-N⁺H₂(R")A⁻, -Q-N(R")-CH₂-CH₂-N⁺R"H₂A, wobei jedes Q für eine chemische Bindung, -CH₂-, -CH₂-CH₂-, -CH₂CH₂CH₂-, -C(CH₃)₂-,-CH₂CH₂CH₂CH₂-, -CH₂C(CH₃)₂-, -CH(CH₃)CH₂CH₂- steht,
R" für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, -CH(CH₃)₂, -CH₂CH₂CH₂H₃, -CH₂CH(CH₃)₂, -CH(CH₃)CH₂CH₃, -C(CH₃)₃, steht und A ein Anion repräsentiert.

7. Verwendung nach einem der Ansprüche 1 bis 6, **dadurch gekennzeichnet, dass** sie mindestens ein aminofunktionelles Silikon der Formel (Si-IIa) 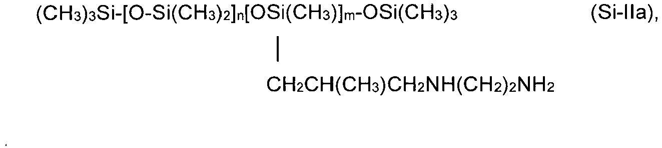 enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

8. Verwendung nach einem der Ansprüche 1 bis 7, **dadurch gekennzeichnet, dass** sie mindestens ein aminofunktionelles Silikon der Formel (Si-IIb) 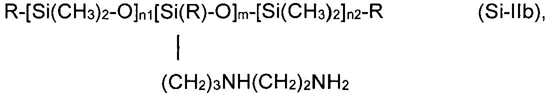 enthalten, worin R für -OH, -O-CH₃ oder eine -CH₃-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

9. Verwendung nach einem der Ansprüche 1 bis 8, **dadurch gekennzeichnet, dass** es mindestens ein weiteres Copolymer B enthält, das 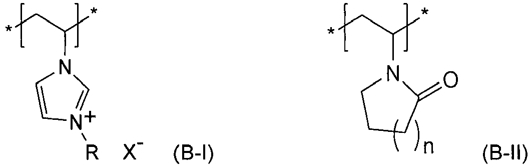 worin
- R eine C₁- bis C₃₀-Alkylgruppe, eine C₁- bis C₄-Aralkylgruppe, eine C₂- bis C₆-Alkenylgruppe oder eine C₂ bis C₆-Hydroxyalkylgruppe bedeutet und
- X⁻ für ein physiologisch verträgliches Anion steht
- n für 1, 2 oder 3 als Anzahl der Methyleneinheiten steht.

10. Verwendung nach einem der Ansprüche 1 bis 9, **dadurch gekennzeichnet, dass** es mindestens ein Acrylat-Polymer C, ausgewählt aus c1) Polyacrylsäure und/oder c2) Copolymeren von Methacrylsäure mit Acrylamidopropansulfonsäure und/oder c3) Copolymeren von Acrylsäure mit Methacrylsäure und Acrylsäureestern und/oder c4) Copolymeren von Acrylsäure mit Methacrylsäure mit Acrylsäureestern und Methacrylsäureestern und/oder c5) Copolymeren von Acrylsäureestern mit Methacrylsäure enthält.

11. Verwendung nach einem der Ansprüche 1 bis 10, **dadurch gekennzeichnet, dass** es mindestens ein Copolymer E, gebildet aus mindestens einem Monomer e1 ausgewählt aus Acrylsäure und/oder Methacrylsäure, und mindestens einem Monomer e2 ausgewählt aus Acrylamid und/oder Methacrylamid und mindestens einem Monomer e3 ausgewählt aus N-substituierten Acrylamiden und/oder Methacrylamiden enthält.

12. Verwendung nach einem der Ansprüche 1 bis 11, **dadurch gekennzeichnet, dass** es mindestens ein Copolymer F, ausgewählt aus f1) Copolymeren von Vinylpyrrolidon mit Methacrylamidopropyltrimethylammoniumchlorid (MAPTAC) und/oder f2) Copolymeren von Vinylpyrrolidon mit Dimethylaminoethylmethacrylat und/oder f3) Copolymeren von Vinylpyrrolidon mit Dimethylaminopropylmethacrylamid und Alkyldimethylpropylmethacrylamidoammoniumsalzen enthält.

13. Verwendung nach einem der Ansprüche 1 bis 12, **dadurch gekennzeichnet, dass** es sich um ein Stylinggel, eine Pumphaarspray, ein Aerosolhaarspray, einen Pumphaarschaum oder ein Aerosolhaarschaum handelt.

14. Verfahren zur temporären Verformung keratinischer Fasern, **dadurch gekennzeichnet, dass** ein kosmetisches Mittel nach einem der Ansprüche 1 bis 13 auf das Haar als Pumphaarspray, Aerosolhaarspray, Pumphaarschaum, Aerosolhaarschaum oder Stylinggel aufgetragen wird und gegebenenfalls mit den Handflächen und/oder den Fingern in das Haar eingearbeitet wird.

## Claims

1. The use of a cosmetic agent containing, in a cosmetically acceptable carrier,
a) at least one copolymer A, which contains at least one structural unit according to formula (I) and at least one structural unit according to formula (II) and at least one structural unit according to formula (III) 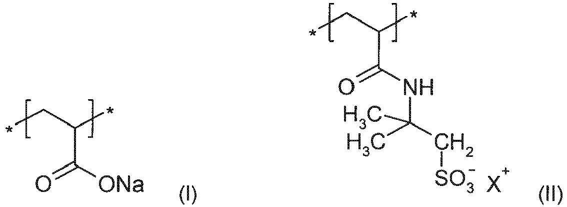 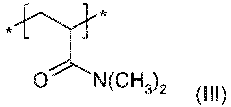 wherein X⁺ represents a physiologically acceptable cation
and
b) at least one silicone oil and/or silicone gum
for temporarily deforming keratin fibers.

2. The use according to claim 1, **characterized in that** said agent contains copolymer(s) A having a molar mass of from 10 to 750 kDa, preferably 25 to 500 kDa, more preferably 50 to 400 kDa and in particular 70 to 250 kDa.

3. The use according to one of claims 1 or 2, **characterized in that** said agent contains, based on the weight of the ready-to-apply agent, from 0.1 to 10 wt.%, preferably 0.5 to 7.5 wt.% and in particular 1 to 5 wt.%, copolymer(s) A.

4. The use according to one of claims 1 to 3, **characterized in that** said agent contains copolymer(s) A which contain from 10 to 90 mol.% (preferably 15 to 85 mol.% and in particular 20 to 80 mol.%) monomers of formula (I) and from 5 to 85 mol.% (preferably 7.5 to 80 mol.%) and in particular 10 to 60 mol.%) monomers of formula (II) and from 5 to 85 mol.% (preferably 10 to 80 mol.% and in particular 15 to 70 mol.%) monomers of formula (III).

5. The use according to one of claims 1 to 4, **characterized in that** at least one silicone of formula Si-I
(CH₃)₃Si-[O-Si(CH₃)₂]ₓ-O-Si(CH₃)₃ (Si-I)
is contained, in which x represents a number of from 0 to 100, preferably 0 to 50, more preferably 0 to 20, and in particular 0 to 10.

6. The use according to one of claims 1 to 5, **characterized in that** said agent contains at least one aminofunctional silicone of formula (Si-II)
R'ₐG₃₋ₐ-Si(OSiG₂)ₙ-(OSiG_{b}R'_{2-b})ₘ-O-SiG₃₋ₐ-R'ₐ (Si-II),
in which:
- G is -H, a phenyl group, -OH, -O-CH₃, -CH₃, -O-CH₂CH₃, -CH₂CH₃, -O-CH₂CH₂CH₃,-CH₂CH₂CH₃, -O-CH(CH₃)₂, -CH(CH₃)₂, -O-CH₂CH₂CH₂CH₃, -CH₂CH₂CH₂CH₃, -O-CH₂CH(CH₃)₂, -CH₂CH(CH₃)₂, -O-CH(CH₃)CH₂CH₃, -CH(CH₃)CH₂CH₃, -O-C(CH₃)₃, or-C(CH₃)₃;
- a represents a number between 0 and 3, in particular 0;
- b represents a number between 0 and 1, in particular 1,
- m and n are numbers whose sum (m + n) is between 1 and 2000, preferably between 50 and 150, n preferably assuming values of from 0 to 1999 and in particular 49 to 149 and m preferably assuming values of from 1 to 2000, in particular 1 to 10,
- R' is a monovalent functional group selected from -Q-N(R")-CH₂-CH₂-N(R")₂, -Q-N(R")₂,-Q-N⁺(R")₃A⁻, -Q-N⁺H(R")₂A⁻ , -Q-N⁺H₂(R")A⁻, -Q-N(R")-CH₂-CH₂-N⁺R"H₂A⁻,
each Q representing a chemical bond, -CH₂-, -CH₂-CH₂-, -CH₂CH₂CH₂-, -C(CH₃)₂-,-CH₂CH₂CH₂CH₂-, -CH₂C(CH₃)₂-, -CH(CH₃)CH₂CH₂-,
R" represents identical or different functional groups from the group -H, -phenyl, -benzyl, -CH₂-CH(CH₃)Ph, of the C₁₋₂₀ alkyl functional groups, preferably -CH₃, -CH₂CH₃,-CH₂CH₂CH₃, -CH(CH₃)₂, -CH₂CH₂CH₂H₃, -CH₂CH(CH₃)₂, -CH(CH₃)CH₂CH₃, -C(CH₃)₃, and A represents an anion.

7. The use according to one of claims 1 to 6, **characterized in that** said agent contains at least one aminofunctional silicone of formula (Si-IIa) 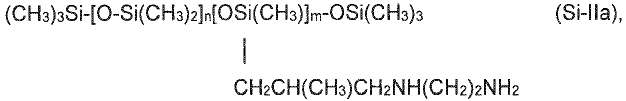 in which m and n are numbers whose sum (m + n) is between 1 and 2000, preferably between 50 and 150, n preferably assuming values of from 0 to 1999 and in particular 49 to 149 and m preferably assuming values of from 1 to 2000, in particular 1 to 10.

8. The use according to one of claims 1 to 7, **characterized in that** said agent contains at least one aminofunctional silicone of formula (Si-IIb) 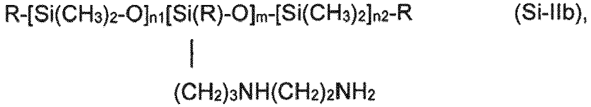 in which R represents -OH, -O-CH₃, or a -CH₃ group and m, n1 and n2 are numbers whose sum (m + n1 + n2) is between 1 and 2000, preferably between 50 and 150, the sum (n1 + n2) preferably assuming values of from 0 to 1999 and in particular 49 to 149 and m preferably assuming values from 1 to 2000, in particular 1 to 10.

9. The use according to one of claims 1 to 8, **characterized in that** said agent contains at least one further copolymer B 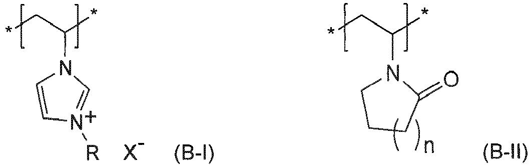 in which
- R is a C₁ to C₃₀ alkyl group, a C₁ to C₄ aralkyl group, a C₂ to C₆ alkenyl group, or a C₂ to C₆ hydroxyalkyl group
- X⁻ represents a physiologically acceptable anion
- n represents 1, 2 or 3 as the number of methylene units.

10. The use according to one of claims 1 to 9, **characterized in that** said agent contains at least one acrylate polymer C selected from c1) polyacrylic acid and/or c2) copolymers of methacrylic acid with acrylamidopropanesulfonic acid and/or c3) copolymers of acrylic acid with methacrylic acid and acrylic acid esters and/or c4) copolymers of acrylic acid with methacrylic acid with acrylic acid esters and methacrylic acid esters and/or c5) copolymers of acrylic acid esters with methacrylic acid.

11. The use according to one of claims 1 to 10, **characterized in that** said agent contains at least one copolymer E formed of at least one monomer e1 selected from acrylic acid and/or methacrylic acid, and at least one monomer e2 selected from acrylamide and/or methacrylamide, and at least one monomer e3 selected from N-substituted acrylamides and/or methacrylamides.

12. The use according to one of claims 1 to 11, **characterized in that** said agent contains at least one copolymer F selected from f1) copolymers of vinylpyrrolidone with methacrylamidopropyltrimethylammonium chloride (MAPTAC) and/or f2) copolymers of vinylpyrrolidone with dimethylaminoethyl methacrylate and/or f3) copolymers of vinylpyrrolidone with dimethylaminopropylmethacrylamide and alkyldimethylpropylmethacrylamide ammonium salts.

13. The use according to one of claims 1 to 12, **characterized in that** the agent is a styling gel, a pump hairspray, an aerosol hairspray, a pump hair mousse, or an aerosol hair mousse.

14. A method for temporarily deforming keratin fibers, **characterized in that** a cosmetic agent according to one of claims 1 to 13 is applied to the hair as a pump hairspray, aerosol hairspray, pump hair mousse, aerosol hair mousse or styling gel and, if necessary, is worked into the hair using the palms of the hands and/or the fingers.

## Revendications

1. Utilisation d'un agent cosmétique, contenant dans un excipient cosmétiquement acceptable
a) au moins un copolymère A, qui contient au moins une unité structurelle selon la formule (I) et au moins une unité structurelle selon la formule (II) et au moins une unité structurelle selon la formule (III) 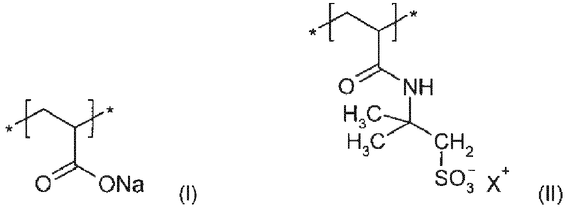 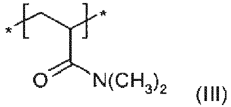 dans laquelle X⁺ représente un cation physiologiquement acceptable et
b) au moins une huile de silicone et/ou de gomme de silicone
pour la mise en forme temporaire de fibres kératiniques.

2. Utilisation selon la revendication 1, **caractérisée en ce qu'**il contient un ou plusieurs copolymère(s) A ayant des masses molaires comprises entre 10 à 750 kDa, de préférence de 25 à 500 kDa, de manière davantage préférée de 50 à 400 kDa et en particulier de 70 à 250 kDa.

3. Utilisation selon une des revendications 1 ou 2, **caractérisée en ce qu'**il contient, par rapport au poids de l'agent prêt à l'emploi, 0,1 à 10 % en poids, de préférence 0,5 à 7,5 % en poids et en particulier 1 à 5 % en poids de copolymère(s) A.

4. Utilisation selon une des revendications 1 à 3, **caractérisée en ce qu'**il contient un ou plusieurs copolymère(s) A, qui contiennent 10 à 90 % en mol (de préférence 15 à 85 % en mol et en particulier 20 à 80 % en mol) de monomères de formule (I) et 5 à 85 % en mol (de préférence 7,5 à 80 % en mol et en particulier 10 à 60 % en mol) de monomères de formule (II) et 5 à 85 % en mol (de préférence 10 à 80 % en mol et en particulier 15 à 70 % en mol) de monomères de formule (III).

5. Utilisation selon une des revendications 1 à 4, **caractérisée en ce qu'**ils contiennent au moins un silicone de formule Si-I
(CH₃)₃Si-[O-Si(CH₃)₂]ₓ-O-Si(CH₃)₃ (Si-I),
dans laquelle x représente un nombre de 0 à 100, de préférence de 0 à 50, de manière davantage préférée de 0 à 20 et en particulier de 0 à 10.

6. Utilisation selon une des revendications 1 à 5, **caractérisée en ce qu'**ils contiennent au moins un silicone aminofonctionnel de formule (Si-II)
R'ₐG₃₋ₐ-Si(OSiG₂)ₙ-(OSiG_{b}R'_{2-b})ₘ-O-SiG₃₋ₐ-R'ₐ (Si-II),
étant donné que :
- G est -H, un groupe phényle, -OH, -O-CH₃, -CH₃, -O-CH₂CH₃, -CH₂CH₃, -O-CH₂CH₂CH₃, CH₂CH₂CH₃, -O-CH(CH₃)₂, -CH(CH₃)₂, -O-CH₂CH₂CH₂CH₃, -CH₂CH₂CH₂CH₃, O-CH₂CH(CH₃)₂, -CH₂CH(CH₃)₂, -O-CH(CH₃)CH₂CH₃, -CH(CH₃)CH₂CH₃, -O-C(CH₃)₃,-C(CH₃)₃ ;
- a représente un nombre compris entre 0 et 3, en particulier 0 ;
- b représente un nombre compris entre 0 et 1, en particulier 1,
- m et n sont des nombres dont la somme (m + n) est comprise entre 1 et 2000, de préférence entre 50 et 150, n prenant de préférence des valeurs allant de 0 à 1999 et en particulier de 49 à 149 et m prenant de préférence des valeurs de 1 à 2000, en particulier 1 à 10,
- R' est un radical monovalent sélectionné parmi -Q-N(R")-CH₂-CH₂-N(R")₂, -Q-N(R")₂, Q-N⁺(R")₃A⁻ , -Q-N⁺H(R")₂A⁻ , -Q-N⁺H₂(R")A⁻ , -Q-N(R")-CH₂-CH₂-N⁺R"H₂A⁻ ,
où chaque Q représente une liaison chimique, -CH₂-, -CH₂-CH₂-, -CH₂CH₂CH₂-, -C(CH₃)₂-, - CH₂CH₂CH₂CH₂-, -CH₂C(CH₃)₂-, -CH(CH₃)CH₂CH₂-,
R" représente des radicaux identiques ou différents du groupe -H, -phényle, -benzyle,-CH₂-CH(CH₃)Ph, des radicaux alkyle en C₁₋₂₀, de préférence -CH₃, -CH₂CH₃,-CH₂CH₂CH₃, -CH(CH₃)₂, -CH₂CH₂CH₂H₃, -CH₂CH(CH₃)₂, -CH(CH₃)CH₂CH₃, -C(CH₃)₃, et A représente un anion.

7. Utilisation selon une des revendications 1 à 6, **caractérisée en ce qu'**ils contiennent au moins un silicone aminofonctionnel de formule (Si-Ila) 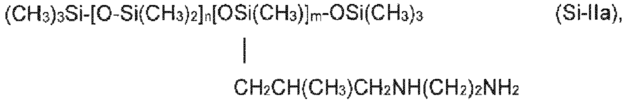 dans laquelle m et n sont des nombres dont la somme (m + n) est comprise entre 1 et 2000, de préférence entre 50 et 150, n prenant de préférence des valeurs allant de 0 à 1999 et en particulier de 49 à 149, et m prenant de préférence des valeurs allant de 1 à 2000, en particulier de 1 à 10.

8. Utilisation selon une des revendications 1 à 7, **caractérisée en ce qu'**ils contiennent au moins un silicone aminofonctionnel de formule (Si-IIb) 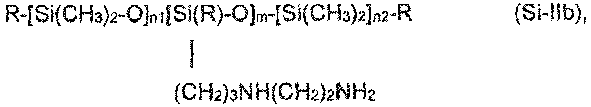 dans laquelle R représente -OH, -O-CH₃ ou un groupe -CH₃ et m, n1 et n2 sont des nombres dont la somme (m + n1 + n2) est comprise entre 1 et 2000, de préférence entre 50 et 150, la somme (n1 + n2) prenant de préférence des valeurs allant de 0 à 1999 et en particulier de 49 à 149, et m prenant de préférence des valeurs de 1 à 2000, en particulier de 1 à 10.

9. Utilisation selon une des revendications 1 à 8, **caractérisée en ce qu'**ils contiennent au moins un copolymère B supplémentaire, 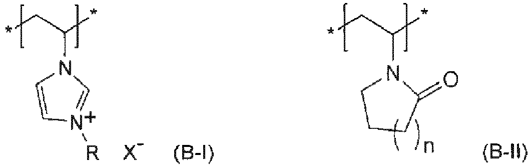 où
- R représente un groupe alkyle en C₁ à C₃₀, un groupe aralkyle en C₁ à C₄, un groupe alcényle en C₂ à C₆ ou un groupe hydroxyalkyle en C₂ à C₆ et
- X- représente un anion physiologiquement acceptable
- n représente 1, 2 ou 3 comme nombre d'unités méthylène.

10. Utilisation selon une des revendications 1 à 9, **caractérisée en ce qu'**il contient au moins un polymère d'acrylate C sélectionné parmi c1) l'acide polyacrylique et/ou c2) des copolymères d'acide méthacrylique avec de l'acide acrylamidopropanesulfonique et/ou c3) des copolymères d'acide acrylique avec de l'acide méthacrylique et des esters d'acide acrylique et/ou c4) des copolymères d'acide acrylique avec de l'acide méthacrylique et des esters d'acide acrylique et des esters d'acide méthacrylique et/ou c5) des copolymères d'esters d'acide acrylique avec de l'acide méthacrylique.

11. Utilisation selon une des revendications 1 à 10, **caractérisée en ce qu'**il contient au moins un copolymère E, formé à partir d'au moins un monomère e1 choisi parmi l'acide acrylique et/ou l'acide méthacrylique, et d'au moins un monomère e2 choisi parmi l'acrylamide et/ou le méthacrylamide et d'au moins un monomère e3 choisi parmi les acrylamides et/ou méthacrylamides N-substitués.

12. Utilisation selon une des revendications 1 à 11, **caractérisée en ce qu'**il contient au moins un copolymère F, choisi parmi f1) des copolymères de vinylpyrrolidone avec du chlorure de méthacrylamidopropyltriméthylammonium (MAPTAC) et/ou f2) des copolymères de vinylpyrrolidone avec du méthacrylate de diméthylaminoéthyle et/ou f3) des copolymères de vinylpyrrolidone avec du diméthylaminopropylméthacrylamide et des sels d'alkyldiméthylpropylméthacrylamidoammonium.

13. Utilisation selon une des revendications 1 à 12, **caractérisée en ce qu'**il s'agit d'un gel coiffant, d'un spray capillaire à pompe, d'un spray capillaire en aérosol, d'une mousse capillaire à pompe ou d'une mousse capillaire en aérosol.

14. Procédé pour la mise en forme temporaire de fibres kératiniques, **caractérisé en ce qu'**un agent cosmétique selon l'une quelconque des revendications 1 à 13 est appliqué sur la chevelure sous forme de spray capillaire à pompe, de spray capillaire en aérosol, de mousse capillaire à pompe, de mousse capillaire en aérosol ou de gel coiffant et est éventuellement incorporé dans la chevelure avec les paumes des mains et/ou les doigts.
